(19) Europäisches Patentamt
European Patent Office
Office européen des brevets

(11) EP 3 862 069 A1

(12) EUROPEAN PATENT APPLICATION

(43) Date of publication:
11.08.2021 Bulletin 2021/32

(21) Application number: 20305110.7

(22) Date of filing: 06.02.2020

(51) Int Cl.:
B01D 61/00 (2006.01)        B01D 61/42 (2006.01)
B01D 67/00 (2006.01)        B01D 69/12 (2006.01)
B01D 71/02 (2006.01)        B01D 71/50 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicants:
• Centre National de la Recherche Scientifique
  75016 Paris (FR)
• École Normale Supérieure
  75005 Paris (FR)
• Sorbonne Université
  75006 Paris (FR)

• Université de Paris
  75006 Paris (FR)

(72) Inventors:
• BOCQUET, Lydéric
  75013 PARIS (FR)
• SIRIA, Alessandro
  75012 PARIS (FR)
• M'BARKI, Amin
  75019 PARIS (FR)
• PEREZ-CARVAJAL, Javier
  75013 PARIS (FR)

(74) Representative: Novagraaf Technologies
Bâtiment O2
2, rue Sarah Bernhardt
CS90017
92665 Asnières-sur-Seine Cedex (FR)

(54) **REVERSE ELECTRO-OSMOTIC FILTRATION SYSTEM AND USES THEREOF**

(57) The present invention relates to a purification/filtration system using reverse electro-osmotic flow through a composite or hybrid membrane element. The invention also relates to a process for purifying an electrolyte solution using such system. The invention further relates to a water purification system, a water desalination system and an implantable artificial kidney, comprising a reverse electro-osmotic filtration system according to the invention.

Exemplary system with composite membrane

FIGURE 1

## Description

### FIELD OF THE INVENTION

The present invention relates to a purification/filtration system using a reverse electro-osmotic flow through a composite or hybrid membrane element. The invention also relates to a process for purifying an electrolyte solution using such system.

### BACKGROUND OF THE INVENTION

The increase of population and the improvement of life quality have made the access to clean water a central problem in many parts of the world. Drinkable water is becoming contaminated with a large variety of pollutants coming from different sources, which range from particles to small size molecules like colorants and pesticides, drugs and hormones. Indeed, hormone spread to water by the primary sectors is becoming one of the most common contaminant nowadays and a kind of contaminant difficult to remove. Only a small amount of these molecules, commonly below the standard detection limits, present an environmental and human health hazard. For instance, the use of pregnancy programs releases to the potential drinkable water estradiol and other hormones that has been associated with sperma quality decrease. These hormones present in farming, fine industry or directly excreted by humans are further incorporated into soils, plants and animals. It has to be considered that more than 70% of human breast cancers are estrogen-dependent, and their development and growth are not only influenced by endogenous estrogens but also likely by environmental estrogen-like endocrine disrupters.

Among water purification methods, purification methods involving the osmosis phenomenon are known. Osmosis is a natural phenomenon which is based on the following principle: when two aqueous solutions with different concentrations of impurities such as salts, are separated by a semi-permeable membrane, that is permeable to water and impermeable to impurities such as salts, pure water will pass from the less concentrated solution to the more concentrated solution.

Specifically, the semi-permeable membrane prevents salts dissolved in water from passing therethrough, such that the transfer of pure water is the only means which enables a concentration balance to be established on both sides of the membrane. A direct consequence of the pure water transfer to the compartment containing the concentrated solution is a rise in the pressure of the solution on the membrane. This pressure is called the osmotic pressure ($P_{osm}$).

Two water treatment technologies make use of the properties of semi-permeable membranes and of the osmosis phenomenon, it is on the one hand the so-called reverse osmosis technology, and on the other hand the so-called direct (forward) osmosis technology.

Currently, the most developed technology is the reverse osmosis technology. It consists in applying to the concentrated solution a pressure much higher than the osmotic pressure to reverse the flux of water molecules through the semi-permeable membrane.

The reverse osmosis technology enables a high purity water to be produced, but it is a technology which implements high pressures, much higher than the osmotic pressure $P_{osm}$, and the energy consumption of which is very significant. For example, desalination of water through reverse osmosis is a known technique in the field of water treatment. However, reverse osmosis desalination involves artificially applying a relatively high pressure and thus entails very high energy consumption. Consequently, the investment costs and operating costs of reverse osmosis facilities are high.

In addition, these processes generally allow to obtain high water flow with low selectivity (low rejection rates), especially when the target is the removal of pollutants in the nanosize range. The low concentration and small size of hormone molecules, for instance, do not only impede detection by routine analytical methods in water pollutants determinations, but also impede the removal of this kind of small organic molecules by general purification methods.

Current commercial seawater reverse osmosis (SWRO) desalination may reach up to 50% recovery of desalinated water but can result in a rejected stream that has 7% salinity with relevant osmotic pressure of 784 psi. Actual desalination operating pressure has to account for pumping and control systems inefficiency that may cause required pumping pressure in excess of 1000 psi.

Most semipermeable polymeric membranes that are adequate for seawater desalination cannot sustain operation or maintain separation efficiency at pressure exceeds 1,100 psi and eventfully fail, a condition that may force the system operator to run the desalination process at a lower desalination recovery ,i.e., of 40% or less to sustain the life of the membrane.

Although the processes and systems already known in the art aim at increasing energy efficiency, a major drawback is that they still do demand a relatively high energy consumption.

Accordingly, there exists a significant need for further improvements in and to reverse osmosis water purification systems. The present invention fulfills these needs and provides further related advantages.

## BRIEF DESCRIPTION OF THE DRAWINGS

**Figure 1** schematically shows an exemplary system according to the present invention, involving a <u>composite</u> reverse osmotic membrane element (30).

**Figure 2** schematically shows an exemplary system according to the present invention, involving a <u>hybrid</u> reverse osmotic membrane element (30).

**Figure 3** schematically shows an exemplary system according to the present invention, involving a <u>composite</u> reverse osmotic membrane element (30) with a porous support (33).

**Figure 4** schematically illustrates an exemplary filtration system according to the invention (a), FE-SEM images of the surface of the reverse osmosis membrane element (b), cross-section (c) and X-ray diffractogram. Scale bar, 5 $\mu$m.

**Figure 5** A) Saturation, B) flow versus salt concentration and intensity C) Intensity vs flow D) current vs intensity.

**Figure 6** illustrates exemplary rejection results using a system according to the invention: (A) Rejection first results, (B) second optimization dye, (C) Hormone under EO.

**Figure 7** Simulation approach: computation of water flow throughout an asymmetric membrane under a voltage drop ($\Delta$V) in an exemplary system according to the invention. Q= water flow through the membrane; Qosm= osmotic flow of water through the membrane at $\Delta$V = 0.

**Figure 8** Optical Microscope image of the surface of a composite GO/PC membrane according to the invention (no cracks are detected in long range).

**Figure 9** BET surface area (analysis of the porosity of the GO membrane in the absence of the PC substrate).

**Figure 10** Pore size distribution of the GO membrane showing a wide distribution from 1.8 nm to 6 nm.

**Figure 11** Nitrogen adsorption BET isotherm of a GO membrane.

**Figure 12** Photography of an exemplary embodiment of a filtration system according to the invention.

**Figure 13** Flow versus concentration and voltage using the bare PC membrane as reservoir separator, and KCl electrolyte solutions at different concentrations.

**Figure 14** Calibration curve: Ru(biPy) concentration versus absorbance at 283 nm.

While the system and process of the present application is susceptible to various modifications and alternative forms, specific embodiments thereof have been shown by way of example in the drawings and are herein described in detail. It should be understood, however, that the description herein of specific embodiments is not intended to limit the application to the particular embodiment disclosed, but on the contrary, the intention is to cover all modifications, equivalents, and alternatives falling within the spirit and scope of the process of the present application as defined by the appended claims.

## DEFINITIONS

Unless defined otherwise, all the technical and scientific terms used herein have the same meaning as those generally understood by one of ordinary skill in the art to which the invention pertains. Generally, the nomenclature used herein and the experiment methods, which will be described below, are those well-known and commonly employed in the art.

To facilitate an understanding of the present invention, a number of terms and phrases are defined below:
As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof "a mixture thereof and such like following a listing, the use of "and/or" as part of a listing, a listing in a table, the use of "etc." as part of a listing, the phrase "such as," and/or a listing within brackets with "e.g.," or i.e., refers to any combination (e.g., any sub-set) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

As used herein, the term "and/or" means any one of the items, any combination of the items, or all of the items with which this term is associated.

As will be understood by the skilled artisan, all numbers, including those expressing quantities of ingredients, properties such as cavity/pore size and zeta potential, experimental conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As used herein, the term "about" can refer to a variation of $\pm 5\%$ of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., concentration values, proximate to the recited range that are equivalent in terms of the functionality of the individual ingredient, the composition, or the embodiment.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

## DETAILED DESCRIPTION OF CERTAIN PREFERRED EMBODIMENTS OF THE INVENTION

Illustrative embodiments are described below. In the interest of clarity, not all features of an actual implementation are described in this specification. It will of course be appreciated that in the development of any such actual embodiment, implementation-specific decisions must be made to achieve the developer's specific goals, such as compliance with system-related and business-related constraints, which will vary from one implementation to another. Moreover, it will be appreciated that such a development effort might be complex and time-consuming but would nevertheless be a routine undertaking for those of ordinary skill in the art having the benefit of this disclosure.

In the specification, reference may be made to the spatial relationships between various components and to the spatial orientation of various aspects of components as the devices are depicted in the attached drawings. However, as will be recognized by those skilled in the art after a complete reading of the present application, the devices, members, apparatuses, etc. described herein may be positioned in any desired orientation. Thus, the use of terms to describe a spatial relationship between various components or to describe the spatial orientation of aspects of such components should be understood to describe a relative relationship between the components or a spatial orientation of aspects of such components, respectively, as the device described herein may be oriented in any desired direction.

The system and process in accordance with the present application overcomes one or more of the above-discussed problems commonly associated with conventional membrane technology and processes. Specifically, the system of the present application uses a reverse electro-osmotic filtration system. This and other unique features of the system are discussed below and illustrated in the accompanying drawings.

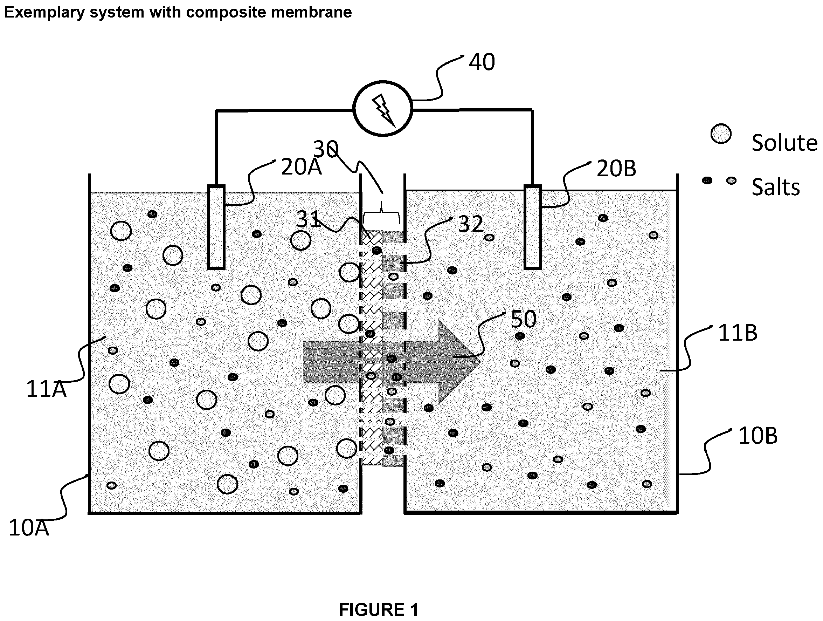

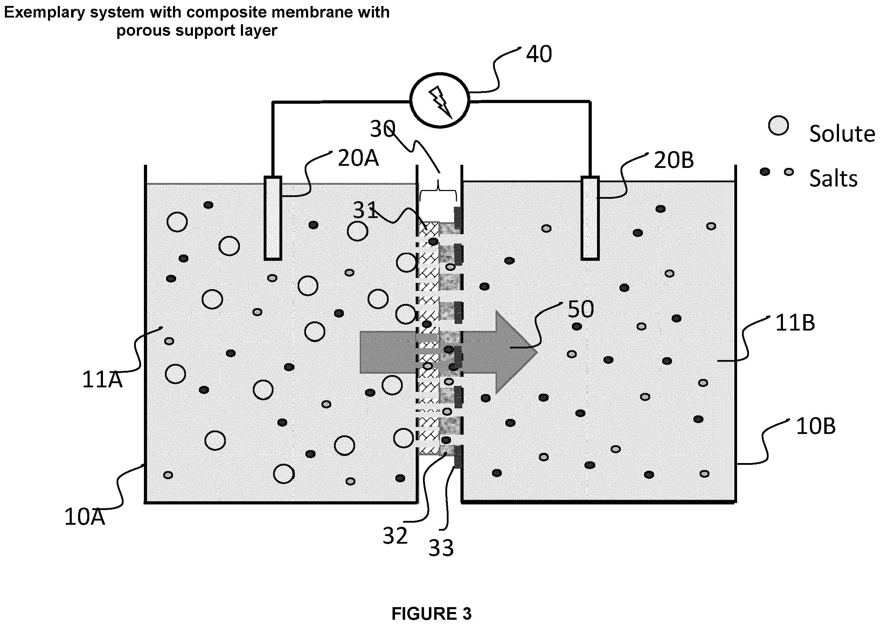

The system and process will be understood, both as to its structure and operation, from the accompanying drawings, taken in conjunction with the accompanying description. Several embodiments of the system are presented herein within FIGS. 1-14. It should be understood that various components, parts, and features of the different embodiments may be combined together and/or interchanged with one another, all of which are within the scope of the present application, even though not all variations and particular embodiments are shown in the drawings. It should also be understood that the mixing and matching of features, elements, and/or functions between various embodiments is expressly contemplated herein so that one of ordinary skill in the art would appreciate from this disclosure that the features, elements, and/or functions of one embodiment may be incorporated into another embodiment as appropriate, unless otherwise described.

In one aspect, the invention provides a reverse electro-osmotic filtration system comprising:

a) a first vessel (10A) intended to receive an electrolyte solution (11A) concentrated in a solute of interest, and comprising a first electrode (20A) in contact with the electrolyte solution (11A) contained in vessel (10A);

b) a second vessel (10B) intended to receive an electrolyte solution (11B) substantially free of, or depleted in, the same solute of interest, and comprising a second electrode (20B) in contact with the electrolyte solution (11B) contained in vessel (10B);

c) a reverse osmosis membrane element (30) separating the two vessels, combining

(i) a semipermeable membrane element (31), and

(ii) a nanoporous membrane element (32) having a surface charge with a | zeta potential | ≥ 5 mV, preferably ≥ 20 mV, most preferably ≥ 50 mV;

wherein the semipermeable membrane element (31) and the nanoporous membrane element (32) having a surface charge may be one and the same element, or two distinct elements;

wherein the semipermeable membrane element (31) is configured to be in contact with the electrolyte solution (11A) of the first vessel (10A), and the nanoporous membrane element (32) having a surface charge is configured to be in contact with the electrolyte solution (11B) of the second vessel (10B);

said reverse osmosis membrane element (30) fulfilling both (i) a function of solute filtration and (ii) a function of electroinducing the flow of the solvent from the electrolyte solution (11A) from vessel (10A) to vessel (10B) through the reverse osmosis membrane element (30);

the electrolyte solutions (11A) and (11B) in vessels (10A) and (10B), respectively, comprising the same polar solvent;
the first and second electrodes (20A) and (20B) being operatively coupled to an electric energy source (40);
wherein the application of an electric field between the first and second electrodes (20A) and (20B) induces a reverse-osmosis flow of the polar solvent of the electrolyte solution (11A) from vessel (10A) to vessel (10B), through the reverse osmosis membrane element (30).

The filtration system according to the invention may be a purification system.

In another aspect, the invention provides a process for purifying an electrolyte solution in a polar solvent, comprising the steps of:

i) providing an electrolyte solution (11A) comprising an undesired solute in a polar solvent;

ii) providing a second electrolyte solution (11B) in the same or different polar solvent as in step i), not comprising the undesired solute;

iii) providing a reverse electro-osmotic filtration system comprising:

a) a first vessel (10A) equipped with a first electrode (20A);

b) a second vessel (10B) equipped with a second electrode (20B); the first and second electrodes (20A) and (20B) being operatively coupled to an electric energy source (40);

c) a reverse osmosis membrane element (30) separating the two vessels (10A) and (10B), combining

(i) a semipermeable membrane element (31), and

(ii) a nanoporous membrane element (32) having a surface charge with a |zeta potential| ≥ 5 mV, preferably ≥ 20 mV, most preferably ≥ 50 mV;
wherein the semipermeable membrane element (31) and the nanoporous membrane element (32) having a surface charge may be one and the same element, or two distinct elements;
wherein the semipermeable membrane element (31) is in contact with the electrolyte solution (11A) of vessel (10A), and the nanoporous membrane element (32) having a surface charge is in contact with the electrolyte solution (11B) of vessel (10B);

said reverse osmosis membrane element (30) fulfilling both (i) a function of solute filtration and (ii) a function of electroinducing the flow of the solvent of the electrolyte solution (11A) from vessel (10A) to vessel (10B) through the reverse osmosis membrane element (30);
the reverse osmosis membrane element (30) being adapted to selectively prevent the undesired solute from diffusing though the reverse osmosis membrane element (30);

iv) placing the electrolyte solution (11A) to be purified in vessel (10A), so that the first electrode (20A) with which it is equipped is in contact with said electrolyte solution (11A);

v) placing a sufficient amount of the second electrolyte solution (11B) in vessel (10B), so that the second electrode (20B) with which it is equipped is in contact with said electrolyte solution (11B);

vi) applying an electric field between the first and second electrodes (20A) and (20B) to induce a reverse-osmosis flow of the polar solvent from vessel (10A) to vessel (10B), through the reverse osmosis membrane element (30) separating the two vessels (10A) and (10B);

thereby allowing the solvent to flow from vessel (10A) towards vessel (10B), while retaining the undesired solute in vessel (10A); and

vii) collecting the purified electrolyte solution (11B) from vessel (10B), which is substantially free of, or depleted in, undesired solute.

As used herein, the term "solute" refers to a liquid or solid material that dissolves in the polar solvent used in electrolyte solutions (11A) and/or (11B).

As used herein, the term "substantially free of a solute" means that $\geq 99\%$, preferably $\geq 99.5\%$, more preferably $\geq 99{,}8\%$, still more preferably $\geq 99{,}9\%$, most preferably 100% of the solute has been removed.

As used herein, the term "concentrated in a solute" means that the feed electrolyte solution (11A) contains a higher concentration of solute than the eluent electrolyte solution (11B).

As used herein, the term "depleted in a solute" means that the eluent electrolyte solution (11B) contains a smaller concentration of solute than the feed electrolyte solution (11A).

As used herein, the term "semipermeable membrane" does not deviate from the conventional meaning of the term in the field of filtration membranes, and refers to a membrane that will allow certain molecules or ions to pass through it by diffusion or man-made generated flux. For example, it may be a size exclusion membrane, an ion exchange membrane, or any other membrane allowing the separation/filtration of particular molecules or ions from a given electrolyte solution (for example semi-permeable membranes based on separation by chemical affinity).

As used herein, the term "electrolyte solution" refers to an electrically conducting solution containing mobile ions, which induces electro-osmose.

As used herein, the term "nanoporous membrane" does not deviate from the conventional meaning of the term in the field of filtration membranes, and refers to a membrane with average pore size $< 1\ \mu$m, preferably $\leq 500$ nm.

As used herein, "zeta potential" when referring to membrane surface charge does not deviate from the conventional meaning of the term in electrochemistry and refers to the potential difference between the membrane surface and the stationary layer of fluid attached to the membrane surface. The zeta potential typically depends from the nature of the membrane surface, and characteristics of the electrolyte solution that is in contact with the membrane surface (e.g., pH, ion concentration, ionic force, ...). The zeta potential may be calculated using the Smoluchowski equation (cf. Equ. 1 infra).

As used herein, |zeta potential| refers to the absolute value of the zeta potential (i.e., the numerical value of the zeta potential without regard to its sign). As will readily be understood from the present disclosure, the invention may be carried out using nanoporous membrane elements bearing a negative or positive surface charge: the zeta potential may be negative or positive, respectively, once the membrane is put into contact with an electrolyte solution.

As used herein, the term "polar solvent" does not deviate from the conventional meaning and from common knowledge/usage in the field. In general, polar solvents include without limitation aprotic solvents having a dielectric constant $\geq 6$ and a dipole moment $\geq 1.50$ D, and protic solvents such as water, alcohols, formic acid, acetic acid, hydrogen fluoride, and ammonia.

The reverse osmosis membrane element (30) may be a composite membrane element, or a hybrid membrane element.

The permeate, from which the excluded solutes have been removed, will exit the reverse osmosis membrane element (30) as the permeate stream (50) and may be collected in vessel (10B). As such, the present invention is also directed to a method of separating a solvent and a solute from a solute-containing electrolyte solution using the filtration system or the purification process according to the invention.

### 1. Composite membrane

An exemplary embodiment is illustrated in Figure 1.

In a variant, the reverse osmosis membrane element (30) may be a composite membrane element, and the reverse electro-osmotic filtration system according to the invention, wherein the semipermeable membrane element (31) and the nanoporous membrane element (32) having a surface charge are two distinct elements combined together to form a two-layer composite asymmetric membrane (30A). Advantageously, said composite asymmetric membrane (30A) may comprise a semipermeable membrane (31) superimposed with a charged nanoporous membrane (32) bearing a surface charge with a |zeta potential| $\geq 5$ mV, preferably $\geq 20$ mV, most preferably $\geq 50$ mV.

As discussed below, the semipermeable membrane element may itself combine a semipermeable membrane and a porous support layer.

1.1. Semipermeable membrane

Semipermeable membranes useable in the context of the present invention may be any semipermeable membrane known in the art. For example, the semipermeable membrane element (31) may be a size exclusion membrane, an ion exchange membrane, or any other membrane allowing the separation/filtration of particular molecules or ions from a given electrolyte solution (for example semi-permeable membranes based on separation by chemical affinity), advantageously a size exclusion membrane or ion exchange membrane. For example, it may be a semipermeable membrane which separates pure water molecules from salts and other impurities: a membrane that have permeability to water and relative impermeability to various dissolved impurities including dissolved salts, organics, bacteria, and pyrogens and other small molecules. As such, it may be a semipermeable membrane conventionally used in water purification technology that uses a semipermeable membrane to remove ions, molecules, and larger particles from contaminated water. It may be a semipermeable membrane for use in water purification or desalination systems, in reverse osmosis systems, such as thin film composite membranes (TFC or TFM). Membranes used in reverse osmosis are, in general, made out of polyamide, chosen primarily for its permeability to water and relative impermeability to various dissolved impurities including salt ions and other small molecules that cannot be filtered.

The porosity of the semipermeable membrane will be adapted to filter out a target solute from electrolyte solution (11A). As such, the membrane porosity will be small enough to prevent the target solute (and any other solute with a particle size greater than the target solute) from passing through the semipermeable membrane (31), while letting the polar solvent and other smaller sized solutes to pass through the semipermeable membrane (31). The porosity of the semipermeable membrane will be adapted depending on the intended application type: microfiltration (50-500 nm), ultrafiltration (1-50 nm), or nano-filtration (≤1 nm). Advantageously, the semipermeable membrane element (31) may have a porosity < 500 nm, preferably < 300 nm, more preferably < 100 nm, most preferably < 50 nm. In a preferred variant, the filtration system is intended for ultrafiltration, and the semipermeable membrane element (31) may have a porosity between 1-50 nm.

Advantageously, the semipermeable membrane may be a nanoporous carbon membrane. Nanoporous carbon membranes include carbon nanotube membranes, nanoporous graphene membranes, and multilayer graphene oxide membranes. All these materials can show similar properties of selective permeability and may be used as molecular sieves in applications involving membrane separation, such as nanofiltration, desalination, etc.
Carbon nanotube membranes consist of two pierced graphene sheets connected by short carbon nanotubes of defined diameter. Their practical application is however limited due to the complexity of their manufacture. Nanoporous graphene membranes consist of a single sheet of graphene with nanopores of defined size. However, it is still difficult to obtain large graphene sheets with a pre-defined pore size and a high pore density. Graphene oxide (GO) membranes are composed of stacked GO nanosheets separated by interconnected nanochannels which form the pores allowing selective permeation through the membrane. They can be produced relatively easily and cheaply by depositing GO solutions onto various supports by spraying, dip coating, spin coating, vacuum filtration, etc., and are currently the most commonly used. Further, GO sheets can converted to graphene-like reduced GO (rGO) sheets, with electrical, thermal, mechanical, and surface properties similar to those of pristine graphene.
Accordingly, in the context of the present invention, the semipermeable membrane may be a carbon nanotube membrane, a nanoporous graphene membrane, or a multilayer GO or rGO membrane, preferably a multilayer GO or rGO membrane, most preferably a multilayer GO membrane.
Advantageously, the semipermeable membrane (31) may be a size exclusion selective membrane composed of stacked graphene oxide flakes. Advantageously, the stacked graphene oxide flakes may form a network of 2D nano-channels with an interlayer spacing between GO flakes ranging from 0.7 - 1.4 nm. Interlayer spacing between stacked graphene oxide flakes may be measured by any suitable method known in the art. For example, it may be measured using XRD.
Advantageously, the nanoporous carbon membrane pore size may range from 0.7 nm to 1.5 nm, preferably ≤ 1.4 nm, more preferably ≤ 1.3 nm, still more preferably ≤ 1.1 nm, and even more preferably ≤ 1.0 nm. The "pore size" when referring to carbonaceous semipermeable membrane, refers to the pore diameter in the case of carbon nanotubes and nanoporous graphene membranes, and to the width of the inter-layer gaps in the case of multilayer GO or rGO membranes. Typically, the nanoporous carbon membrane may be from 0.05 $\mu$m to 1 $\mu$m thick, preferably of from 100 to 500 nm thick, in the case of a carbon nanotube membrane. In the case of a multilayer GO or rGO membrane, the carbon membrane may comprise at least 2, preferably at least 3, and up to 300 layers of GO or rGO sheets, and may be from 0.05 to 20 $\mu$m thick, for example from 0.05 to 15 $\mu$m thick, from 0.05 to 10 $\mu$m thick, from 0.05 to 5 $\mu$m thick, or from 0.05 to 1 $\mu$m thick. For example, the carbon membrane may be about 0.1 $\mu$m thick.

In another exemplary variant, the semipermeable membrane may be an ion exchange membrane, such as anion-exchange membranes (e.g., OH$^-$, Cl$^-$ transport) or cation-exchange membranes (e.g., H$^+$, Na$^+$, K$^+$ transport). The semipermeable membrane may be placed directly on the nanoporous membrane element (32) with a surface charge, or may be first placed on another porous support layer (33) (for ease of manufacture and/or handling, for example) before being placed on the nanoporous membrane element (32) with a surface charge. The porous support layer (33) should have a much greater pore size, to allow the permeation of the flow of the polar solvent of the electrolyte solution (11A) from vessel (10A) to vessel (10B), through the reverse osmosis membrane element (30). As such, the porous support layer, when it is used, preferably has a pore size at least X5, X10, or greater, than the pore size of the semipermeable membrane element (32), and a thickness of from 30 to 300 $\mu$m, more preferably of from 100 to 200 $\mu$m. The porous support layer (33) may be made of a neutral (no surface charge) non-reactive polymeric material, for instance a fluoropolymer such as polyvinylidene fluoride (PVDF) or polytetrafluoroethylene (PTFE), or mixed cellulose ester or cellulose acetate. It can also be made of a porous ceramic material such as an alumina, or silica based porous ceramic, by way of non-limitative examples.

When the semipermeable membrane (31) is placed on a porous support layer (33), the resulting combined filtration membrane can be used in any orientation, i.e. either the semipermeable membrane face, or the porous support face can be contacted with the nanoporous membrane element (32) with a surface charge. Combined filtration membranes where the semipermeable membrane (31) is placed on both sides of the porous support layer (33), or alternatively placed between two porous support layers can also be used, as well as multilayered combined filtration membranes alternating two or more layers of semipermeable membrane (31) with two or more layers of porous support (33).

Advantageously, the semipermeable membrane may be preferably adapted to achieve solute rejection of 98.0% or more, preferably $\geq$ 98.5%, more preferably $\geq$ 99.0%, still more preferably $\geq$ 99.5%, most preferably 100% or about 100% solute rejection.

Naturally, the semipermeable membrane porosity will be adapted to the solute that is intended to be filtered out.

### 1.2. Nanoporous membrane having a surface charge

Nanoporous membranes useable in the context of the present invention may be any membrane having a surface charge known in the art, which is made nanoporous, or any nanoporous membrane wherein at least part of the inner surface of the nanochannel(s) of the membrane is essentially formed of at least one material having a suitable surface charge.

In one preferred embodiment, at least part of the inner surface of the nanochannels is essentially formed of or coated with at least one material having a suitable surface charge. Advantageously, the nanochannels may preferably be entirely formed of or coated with at least one material having a suitable surface charge.

As used herein, "at least part of the inner surface of the nanochannels" refers to the fact that the inner surface of the nanochannels may comprise one or more sections essentially formed of a material having a suitable surface charge, or that the entirety of the inner surface is essentially formed of at least one material having a suitable surface charge. Said section(s) may be regular or irregular, intermittent or non-intermittent and/or in the form of a single layer or multi-layers. Preferably, the total inner surface of the nanochannels is essentially formed of at least one material having a suitable surface charge.

Nanoporous membranes useable in the context of the present invention may have a positive or negative surface charge.

When the nanoporous membrane carries a positive surface charge, the application of an electric field between electrodes (20A) and (20B), where electrode (20B), in contact with the electrolyte solution (11B) of the second vessel (10B) is positively charged, will induce the flow of anions in the electrolyte solution (11B) from the positively charged pore surface of the nanoporous membrane element (32) towards electrode (20B). This anion flow concomitantly induces the flow of the polar solvent from the first vessel (10A) towards the second vessel (10B), through the reverse osmosis membrane element (30).

Conversely, when the nanoporous membrane carries a negative surface charge, the application of an electric field between electrodes (20A) and (20B), where electrode (20B), in contact with the electrolyte solution (11B) of the second vessel (10B) is negatively charged, will induce the flow of cations in the electrolyte solution (11B) from the negatively charged pore surface of the nanoporous membrane element (32) towards electrode (20B). This cation flow concomitantly induces the flow of the polar solvent from the first vessel (10A) towards the second vessel (10B), through the reverse osmosis membrane element (30).

Advantageously, the nanoporous membrane, bearing a positive or negative surface charge, may bear a surface charge with a |zeta potential| $\geq$ 5 mV, preferably $\geq$ 20 mV, most preferably $\geq$ 50 mV.

Examples of materials bearing a surface charge suitable for the nanoporous membrane element of the invention include materials essentially formed of titanium oxide, boron nitride, $SiO_2$, polyethersulfone, polycarbonate, anodic aluminum oxide (anodic alumina), hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes. $Ni_2$dobdc and $Mg_2$dobdc are known metal organic frameworks with dobdc ligands, and Ni or Mg metal sites, respectively. Advantageously, materials bearing a surface charge suitable for the nanoporous membrane element of the invention include materials essentially formed of titanium oxide, boron nitride, $SiO_2$, polyethersulfone,

polycarbonate, anodic aluminum oxide; preferably titanium oxide, boron nitride, $SiO_2$, polycarbonate, and anodic aluminum oxide.

By titanium oxide is meant any type of titanium oxide, for example titanium (IV) oxide or titanium dioxide, and mixtures of two or more thereof. These titanium oxides may be in different solid polymorphous forms, in particular in amorphous form or in crystalline form. In respect of titanium dioxide ($TiO_2$), the rutile or anatase crystalline form type is chiefly used, preferably the anatase form.

As used herein, "essentially formed of titanium oxide, boron nitride, etc..", refers to a material formed of titanium oxide, boron nitride, etc., which may include minority element(s) such as impurities.

The physicochemical properties of materials such as titanium oxide, boron nitride, anodic aluminium oxide, $SiO_2$, can generally be modulated and amplified by doping or functionalisation i.e. by inserting metallic chemical elements on the surface or in the core of the material network, such as iron, silver, vanadium, gold, platinum, niobium, tungsten, or non-metallic elements such as nitrogen, sulfur, carbon, hydrogen, boron, phosphorus, or different chemical compounds of the silane, amine or other organic families, preferably in small amounts.

For example, the nanoporous membrane material may be titanium oxide, which may be doped on the surface or in the core of its crystalline network by inserting metallic chemical elements such as iron, silver, vanadium, gold, platinum, niobium, tungsten, or non-metallic elements such as nitrogen, sulfur, carbon, hydrogen, boron, phosphorus, or different chemical compounds such as silanes or amines, preferably in an amount of between 0.5 and 10 weight % and more preferably between 1 and 5 weight %.

Nanoporous membranes useable in the context of the pressent invention may be carried by a nanoporous or pierced mechanical substrate on which at least one material having a suitable surface charge is deposited. For example, said nanoporous membranes may be composed of a flexible polymer membrane on which a layer of at least one material having a suitable surface charge, such as $TiO_2$, is deposited.

A membrane comprising nanochannels having an inner surface essentially formed of or coated with at least one material having a suitable surface charge, such as ceramic membranes (e.g., titanium oxide, anodic aluminium oxide, $SiO_2$) can be obtained directly by anodizing metallic foil (e.g., Ti, Al or Si) (cf. Progress on free-standing and flow-through TiO2 nanotubes membranes, Guohua Lin, Kaiying Wang, Nils Hoivik, Henrik Jakobsen, Solar Energy Materials & Solar Cells, 98, 2012, pp 24-38; TiO2 nanotubes synthesis and applications, Poulomi Roy, Steffen Berger, Patrick Schmuki, Angewandte Chemistry Int. Ed, 50, 2011, pp 2904-2939). Other techniques such as sol-gel techniques in the presence of block copolymers or grafted copolymers also allow the synthesis of ceramic membranes, such as $TiO_2$ membranes, with regular, oriented nanochannels (cf. Jung Tae Park, Won Seok Chi, Sang Jin Kim, Daeyeon Lee & Jong Hak Kim, Scientific Reports 4:5505, Nature, 2014). With this method, it is also possible to modulate the morphological parameters, length, width and asymmetry of through nanochannels. In addition, powder and sintering technology can be used to obtain very thin ceramic membranes, such as titanium oxide membranes having regular, controlled through nanochannels. Said membranes can also be obtained with different deposition techniques via CVD (Chemical Vapour Deposition), ALD (Atomic Layer Deposition) or HiPIMS (High Power Impulse Magnetron Sputtering), e.g. on nanoporous substrates having preformed morphology.

Alternatively, the surface of the negatively or positively charged material may be chemically modified to enhance the negative or positive charge, respectively, naturally present on the material after it is put into contact with an electrolyte solution, at a given pH. In other words, the surface of the charged nanoporous membrane (32) may be chemically modified to enhance the nanoporous membrane surface charge. The chemical modification may be effected by chemical vapour deposition, atomic layer deposition, sol-gel coating or dip-coating. Advantageously, the chemical modification may be effected on the surface of the nanoporous membrane pore walls. For example, the charged nanoporous membrane (32) may be obtained from a polycarbonate membrane having a porosity < 500 nm, preferably < 300 nm, more preferably < 200 nm; the inner pore walls of which have been chemically modified by dip-coating the polycarbonate membrane in an aqueous solution of polydopamine. At pH=7, polycarbonate bears a negative surface charge, which is enhanced when the polycarbonate surface is coated with polyamine.

In another alternative, when the surface of the membrane material carries ionizable functional groups, the surface charge of the membrane may be modulated using pH. For example, for membrane materials such as $TiO_2$, $SiO_2$, $Al_2O_3$ bearing -OH groups at the surface, driving the pH of the electrolyte solution in contact with the membrane to basic values (pH = 8-14) will deprotonate the -OH groups, thereby enhancing the negatively charged surface. Variations in pH may thus be used to modulate/control the zeta potential at the membrane surface. In fact, depending on the pH of the electrolyte solution with which a nanoporous membrane element (32) is in contact, the surface charge of the same membrane can be positive or negative. For example, a nanoporous membrane element (32) essentially formed of $TiO_2$ car bear a negative surface charge at pH $\geq$9, and a positive surface charge at pH $\leq$ 6.

Without wishing to be bound by any particular theory, it is proposed that the nanochannels of material bearing a negative surface charge, having regard to their type, size and physicochemical properties, in particular their surface charge density in the order of:

Titanium dioxide: ~ - 100 mC/m$^2$ (at pH $\geq$9)
Boron nitride: ~ - 1 C/m$^2$ (at pH $\geq$10)
Silicon dioxide: ~ - 10 mC/m$^2$ (at pH $\geq$ 6)
Polycarbonate: ~ - 10 mC/m$^2$ (at pH $\geq$ 5)

promote the passing of cations (i.e., ions having opposite charges to the material's surface charge) via an electro-osmosis nanofluid phenomenon generated by the application of an electric field. The flow of cations from the Debye layer at the interface of the negatively charged membrane surface, which is globally electrically charged since it contains anion and cation imbalance under the effect of the surface charges, induces in turn the flow of polar solvent in the same direction as the cation flow. Thus, under the application of a suitable electric field, the polar solvent flows through the reverse osmosis membrane element (30) from vessel (10A) to vessel (10B). Since the reverse osmosis membrane element (30) comprises a semipermeable membrane element (31), which is selective for one or more particular solute(s) present in the electrolyte solution (11A), the flow of cations generated in the pores of the nanoporous membrane (32) induces the flow of the electrolyte solution (11A) from vessel (10A) to vessel (10B), while preventing the solute(s) from passing through (the solute(s) get filtered out and stay in vessel (10A)).

Conversely, it is proposed that the nanochannels of material bearing a positive surface charge, promote the passing of anions (i.e., ions having opposite charges to the material's surface charge) via an electro-osmosis nanofluid phenomenon generated by the application of an electric field.

In both cases (cation or anion flow), the flow of polar solvent is induced in the same direction as the cation/anion flow generated upon application of a suitable electric field between electrodes (20A) and (20B).

The membrane surface charge may be measured using any suitable method known in the art. For example, the zeta potentials of the nanoporous membranes may be figured out using an electrokinetic analyser.

The following equation can be implemented to estimate the zeta potential values on the membrane's surface:

$$I_{stream} = -\left(\frac{\varepsilon\zeta}{\eta}\right) A_p \left(\frac{\Delta P}{L}\right) \qquad \textbf{(Equ. 1)}$$

where:

$\varepsilon$ is the dielectric permittivity of the solvent (e.g., water),
$\zeta$ is the zeta potential,
$\eta$ is the viscosity of the solvent (e.g., water),
$A_p$ is the total cross section surface area of all the pores in the membrane exposed to the electrolyte solution,
$\Delta P$ is the pressure drop across the length L of the pores, and
$I_{stream}$ is the electric streaming current.

The length of the pores L may correspond to the thickness of the nanoporous membrane, when nanoporous membranes with a porosity with low tortuosity are used (e.g., nanoporous anodic alumina which have most cylindrical pores). For nanoporous membranes having pores of significant tortuosity, calculations of permeability relative to the applied pressure allows to determine the total pore length by applying a relevant tortuosity factor.

The zeta potential of the membrane surfaces can be measured at different pH values (acidic, neutral and basic conditions) by changing the pH of the electrolyte solution using appropriate concentrations of a suitable acid (e.g., HCl, ) or base (e.g., KOH, NaOH, etc...). Preferably, the acid/base will be selected for compatibility with the ions present in the electrolyte solution used in the process/system according to the invention. For example, when a KCl electrolyte solution is used, KOH may be used as base to adjust the pH.

For example, if one wants a minimum 5 mV of |zeta potential| at the membrane surface, a membrane material carrying a high surface charge may be selected, which allows to maintain a high zeta potential at the surface when the membrane is in contact with the electrolyte solution.

Porosity - nanoporous membrane

The nanoporous membrane element (32) preferably has a porosity greater than that of the semipermeable membrane element (31): the solute filtering function is carried out by the semiperleable element (31), while the function of the nanoporous membrane element (32) is to promote reverse osmotic flow of the solvent upon application of an appropriate electric field between electrodes (20A) and (20B).

Advantageously, the nanoporous membrane element (32) bearing a positive or negative surface charge may have a porosity < 500 nm, preferably < 300 nm, more preferably < 100 nm, most preferably < 50 nm. Preferably, in the present invention, the mean diameter of the nanochannels of the nanoporous membrane element (32) may be between 1 and 500 nm, preferably between 1 and 300 nm, more preferably between 10 and 100 nm, most preferably between 10 and 50 nm.

As used herein, the term "mean diameter" refers to the inner mean diameter of a nanochannel. The nanochannel may have nanotubular, conical asymmetric, neck or perforated base morphology. If the nanochannel has nanotubular morphology i.e. of circular cross-section, the mean diameter corresponds to the inner diameter of the circular cross-section. If the nanochannel has conical asymmetric, neck or perforated base morphology, or an oval or irregular cross-section, the mean diameter corresponds to the mean of the smallest and largest inner diameter. The mean diameter of the nanochannels may be measured using means known to persons skilled in the art. For example, the mean diameter can be measured by scanning electron microscopy or transmission electron microscopy. Advantageously, the nanochannels contained in the nanoporous membrane element (32) may have homogeneous diameters. If, on one same membrane, the nanochannels do not all have homogeneous diameters, the mean diameter will correspond to the mean of the mean diameters of all the nanochannels.

Advantageously, in the present invention, the nanochannels have nanotubular, conical asymmetric, neck or perforated base morphology, preferably said nanochannels have conical asymmetric morphology.

The morphological parameters of the nanoporous membranes useable in the context of the invention may be assessed using conventional techniques, including scanning electron microscopy (SEM), atomic force microscopy (AFM), confocal scanning laser microscopy (CSLM) and transmission electron microscopy (TEM). Alternatively, or additionally, X-ray computed microtomography (micro-CT), nuclear magnetic resonance (NMR), spin-echo small-angle neutron scattering (SESANS) and/or magnetic small-angle neutron scattering (MSANS) may be used (these techniques are generally faster and more complete techniques, as they can give a 3D (volume) analysis). With these techniques, morphological parameters of the nanoporous membranes according to the invention may be assessed, such as pore size, pore size distribution, surface roughness, molecular weight cutoff and thickness.

Pore size represents the dimensions of the pores, which are channels of a variable cross-section. The distance between two opposite pore walls is used as the pore size for simple geometries (typically: diameter of cylindrical pores for pore size >2 nm, width of slit-shaped pores for pore size <2 nm). If the pores have irregular shapes, some averaging is made to report an average pore size. Methods for measuring pore size, average pore size, and pore size distribution of porous materials are well-known in the art (cf. ISO 15901 norm for example), including some statistical analysis using a model such as nonlinear optimization and Monte Carlo integration for materials where the pores do not all have the same size and/or geometry.

Nanoporous membranes useable in the context of the invention may be symmetric or asymmetric. As used herein, "asymmetric" when referring to a nanoporous membrane of the invention, means that the nanoporous membrane pore size distribution is not uniform across the membrane thickness. Conversely, symmetrical membranes have uniform pore size distribution across the membrane thickness. Typically, for asymmetric membranes, a very thin dense surface layer is present acting as a functional layer on top of a porous sublayer with a specific pore diameter. An asymmetric membrane consists, for example, of a $0.1\text{-}1\text{-}\mu\text{m}$-thick skin layer (the selective barrier) on a highly porous $100\text{-}200\text{-}\mu\text{m}$-thick substructure. The pore size of the porous sublayer may be as low as $\leq 1$ nm and as high as 500 nm, the pore size range defining the type of application for which the asymmetric membrane may be used: microfiltration (50-500 nm), ultrafiltration (1-50 nm), and nano-filtration ($\leq 1$ nm). The pore size and its distribution may be determined by numerical analysis of pore dimensions observed in electron micrographs of the membrane cross section. The aforementioned pore size numerical values represents the arithmetic mean of the distribution of pore sizes observed by scanning electron microscopy (SEM) over the membrane cross section.

Advantageously, the mean cross-section of the nanoporous membrane nanochannels and their specific, regular through-morphology promote good diffusion of the solution through the membrane. Therefore, the nanoporous membrane element (32) sets itself clearly apart from the semipermeable membrane element (31), via its nanochannels that potentially allow the circulation both of water molecules and of ions, since each of the nanochannels advantageously has a cross-section larger than the size of these molecules.

2. Hybrid membrane

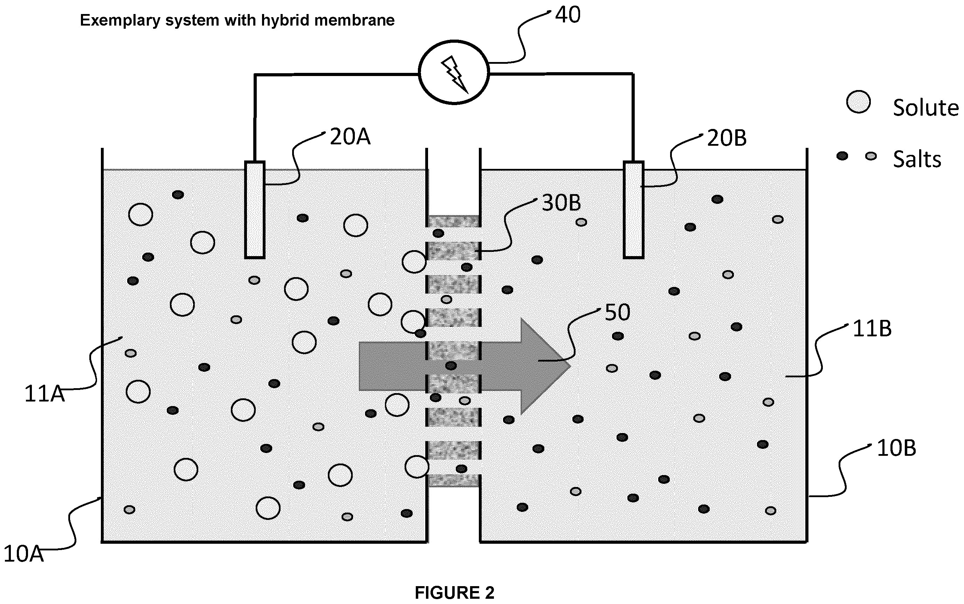

An exemplary embodiment is illustrated in Figure 2.

In another variant, the reverse osmosis membrane element (30) may be a hybrid membrane element, wherein the semipermeable membrane element (31) and the nanoporous membrane element (32) having a surface charge are one and the same element to form a single hybrid membrane (30B). Advantageously, the hybrid membrane (30B) may have a predetermined pore size and zeta potential, adapted to the target solute(s) to be concentrated/depleted or eliminated/filtered out. Advantageously, the single hybrid membrane (30B) may have a porosity adapted to filter out a target solute from electrolyte solution (11A), and a surface charge on the hybrid membrane inner pore walls surface with a |zeta potential| $\geq 5$ mV, preferably $\geq 20$ mV, most preferably $\geq 50$ mV. For example, the porosity of the hybrid membrane will be small enough to prevent the target solute (and any other solute with a particle size greater than the target solute) from passing through the hybrid membrane (30B), while letting the polar solvent and other smaller sized solutes to pass through the hybrid membrane (30B). The porosity of the hybrid membrane (30B) will be adapted depending on the intended application type: microfiltration (50-500 nm), ultrafiltration (1-50 nm), or nano-filtration ($\leq$1 nm). Advantageously, the hybrid membrane (30) may have a porosity < 500 nm, preferably < 300 nm, more preferably < 100 nm, most preferably < 50 nm. In a preferred variant, the filtration system is intended for ultrafiltration, and the hybrid membrane (30B) may have a porosity between 1-50 nm.

The hybrid membrane element may be a nanoporous membrane having a suitable (positive or negative) surface charge, as described generally and in any variant above including preferred and advantageously variants, with a porosity adapted to eliminate/filter out a target solute from electrolyte solution (11A), as described in the paragraph immediately above. The description part regarding the nanoporous membranes is not reproduced here for sake of conciseness, but it will be understood that it is applicable mutatis mutandis to the hybrid membrane element.

Advantageously, the hybrid membrane (30B) may be a nanoporous membrane essentially formed of a material having a surface charge, positive or negative, with a |zeta potential| $\geq$ 5 mV, preferably $\geq$ 20 mV, most preferably $\geq$ 50 mV, such as $TiO_2$, boron nitride, $SiO_2$, polyethersulfone, polycarbonate, anodic aluminum oxide, hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes; preferably $TiO_2$, BN, $SiO_2$, polycarbonate, anodic alumina, hydrotalcite, Ni-Fe layered double hydroxide, $Ni_2$dobdc, $Mg_2$dobdc, cellulose or polyelectrolyte layers polymer membranes; most preferably $TiO_2$, BN, anodic alumina, $SiO_2$, or polycarbonate.

Advantageously, the hybrid membrane (30B) may be a size exclusion selective membrane coated on its inner pore walls with a material having a surface charge, positive or negative, with a |zeta potential| $\geq$ 5 mV, preferably $\geq$ 20 mV, most preferably $\geq$ 50 mV, such as $TiO_2$, boron nitride, $SiO_2$, polyethersulfone, polycarbonate, anodic aluminum oxide, hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes; preferably $TiO_2$, BN, $SiO_2$, polycarbonate, anodic alumina, hydrotalcite, Ni-Fe layered double hydroxide, $Ni_2$dobdc, $Mg_2$dobdc, cellulose or polyelectrolyte layers polymer membranes; most preferably $TiO_2$, BN, anodic alumina, $SiO_2$, or polycarbonate.

Vessels (10A) and (10B)

In the present invention, any reverse osmosis membrane element (30) that separates vessels (10A) and (10B) can be used without any particular limitation as long as it does not to allow a solute to permeate therethrough and mainly allows a solvent to permeate therethrough. Advantageously, the reverse osmosis membrane element (30) may be preferably adapted to achieve solute rejection of 98.0% or more, preferably $\geq$ 98.5%, more preferably $\geq$ 99.0%, still more preferably $\geq$ 99.5%, most preferably 100% or about 100% solute rejection.

For carrying out the process of the invention one can use any separation device/system comprising two compartments separated by the reverse osmosis membrane element (30). The mixture to be separated is placed in a first compartment, (vessel (10A)) and the extracted organic compound is recovered in the second (vessel (10B)).

In the present invention, the reverse electro-osmotic filtration/purification system may be performed in a batch or continuous manner in order to maximize its effect. For example, the reverse electro-osmotic filtration/purification system according to the present invention may be configured in plural numbers. In other words, the unit "vessel (10A)-reverse osmosis membrane element (30)-vessel (10B)" may be configured in multiple stages.

In one exemplary variant, provision could be made so that the reverse electro-osmotic filtration/purification system comprise N vessels (10) and N-1 reverse osmosis membrane elements (30), N being an integer. For example, N may range between 3 and 100, more particularly between 3 and 50. In this multi-vessel device, the vessels and reverse osmosis membrane elements may be such as defined above. The assembly may therefore be formed of alternating vessels (10) alternately containing an electrolyte solution concentrated in a solute of interest and a lesser concentrated electrolyte solution, separated from one another by reverse osmosis membrane elements (30).

Polar solvent

The polar solvent may advantageously be a solvent capable of generating acidic ions. For example, the polar solvent may be water, an alcohol such as methanol or ethanol, a hydroalcoholic mixture, ammonia, acetone, or acetonitrile.

Electrolyte solutions (11A) and (11B) and solutes

Electrolyte solutions useable in the context of the present invention may be any electrolyte solutions containing at least one solute of interest/undesired solute, in a polar solvent as defined herein.

For example, the solvent may be water, or an aqueous solution.

In a preferred variant, the electrolyte solutions may be aqueous solutions comprising electrolytes. The electrolytes may be of any chemical type insofar as they are dissolved in the solution in the form of charged ions. Preferably, these ions derive from dissolved salts such as NaCl, KCl, $CaCl_2$ and $MgCl_2$. The electrolyte solutions may be synthetic solutions; natural solutions such as fresh water from lakes or rivers, underground waters, brackish waters, seawater, industrial production waters, oil production waters or biological solutions/fluids.

Examples of solute-containing aqueous solutions to be used as electrolyte solutions according to the present invention include seawater, brackish water, cellular metabolites, reaction products, biological fluids, etc., wherein the cellular metabolites are intended to include cultures of animal cells, plant cells or microorganisms, their primary metabolites, secondary metabolites, in vitro secreted proteins, biotransformations, etc.

Examples of reaction solutions include chemical reaction products and enzymatic reaction products.

Examples of microorganism primary metabolites include organic acids (e.g., acetic acid, propionic acid, butyric acid, lactic acid, succinic acid and the like), alcohols (e.g., ethanol, butanol and the like), hexanes, amino acids (e.g., lysine, tryptophan and the like), vitamins, polysaccharides, and the like, but is not limited thereto.

Examples of microorganism secondary metabolites include antibiotics (e.g., penicillin and the like), enzyme inhibitors, physiologically active substances (e.g., taxol and the like), and the like, and examples of the in vitro secreted proteins of the microorganisms include enzymes such as amylases, cellulases or the like, insulins, interferons, monoclonal antibodies, and the like. In addition, the biotransformations of the microorganisms are substances produced by using microorganism or enzymes and examples thereof include steroids and the like, but are not limited thereto.

Examples of biological fluids include blood, blood serum, blood plasma, urine, saliva, tears, dialysis fluids, intestinal contents, parenteral nutrition solutions, seminal plasma, and cerebrospinal fluid.

Preferably, said electrolyte solutions may be aqueous solutions comprising a solute selected from among alkaline halides or alkaline-earth halides, preferably selected from among NaCl, KCl, $CaCl_2$ and $MgCl_2$, more preferably the solute is NaCl. The solute may be selected from solid particles, organic or inorganic small molecules such as dye complexes (e.g., Tris(2,2'-bipyridyl)dichlororuthenium (II) hexahydrate), biomolecules such as hormones (e.g., testosterone), proteins, polysaccharides, polynucleotides, polypeptides, enzymes or antibodies, pollutants (for example from waste water, industrial production waters, or landfill leachates), metabolic waste products, or salts/ions. When the solute of interest to be concentrated is a solid, it is preferably a material which is easily crystallized depending on temperature and pH and which is not highly viscous even at high concentrations.

In the present invention, the solute may be salt (or liquid), and the solvent may be water. For example, the electrolyte solution (11A) may be sea water, the solute is NaCl and the process is water desalination. As such, the present invention is also directed to a water desalination system comprising a reverse electro-osmotic filtration system according to the present invention, in any variant described herein.

The present invention is also directed to a water purification system comprising a reverse electro-osmotic filtration system according to the present invention, in any variant described herein. For example, the water pollutant to be filtrated may include endocrine disruptors, hormones, pesticides, and/or dyes

More precisely, in one aspect, the invention is concerned with a method for purifying a liquid comprising water and impurities which implements electro-reverse osmosis technique. By purifying water, water purification, it is generally meant any operation consisting in treating a water containing impurities at an initial content such that at the end of this operation, the final content of impurities is lower than the initial content. By impurity, it is meant any element, molecule, ion, or other, different from the elements constituting pure water, that is $H_2O$, $OH^-$, and $H^+$. This purification method may be, for example, a method for desalinising for example sea water, a method for treating industrial production waters, or a method for treating landfill leachates.

Accordingly, in one aspect, this patent application promotes a methodology for recovering potable water from vast sources of saline water, particularly surface water comprising sodium chloride of various concentrations. Applications range from less than 1% to 20% salinity, for example in the following salinity water application: brackish water of 0.5%-3.5% salinity, seawater of 3.5-4.5% salinity and brine water of 5%-20% salinity.

In the present invention, the solute-containing electrolyte solution may be a biological fluid, preferably dialysis fluids. As such, the present invention is also directed to an implantable artificial kidney comprising a reverse electro-osmotic filtration system according to the present invention, in any variant described herein.

The underlying principle of the invention is that the reverse osmosis membrane element (30) (a single hybrid membrane), or at least part of it (composite membrane comprising a semipermeable membrane element (31) and a nanoporous membrane element (32)), bears a sufficient surface charge (which may be positive of negative) to induce a zeta potential at the membrane/electrolyte solution interface, that is sufficiently high to induce the flow of anions or cations, respectively, through the nanochannels of the nanoporous membrane, upon application of a suitable electric field between electrodes (20A) and (20B). This phenomenon is governed by the same principles of zeta potential applied to colloidal particle suspensions.

In the case of colloidal suspensions, the fact that many organic and mineral colloidal particles possess a negative charge in aqueous environments causes them to repel one another and maintain the stable state of dispersion that characterizes them. Electrochemical dispersion of colloidal particles has been studied for many years. Several models, such as the double-layer and the DLVO theories, have been developed to explain the stability of colloids. The double-layer model predicts that when suspended particles exist in a liquid phase an inner, dense layer at the surface of each particle, consisting of ions in solution attracted by the charge of the particle, exhibits a charge of polarity opposite to the natural charge of the particle itself under the physical and chemical conditions of the suspension. An outer layer of opposite polarity, also consisting of ions in solution, is diffused within a given distance from the surface of the particle. The net potential between the two layers, normally referred to in the art as the zeta potential, produces a repulsion which counteracts van der Waals forces of attraction between the particles. If the outer layer is diffused over a sufficiently wide radius, thereby increasing the effect of the zeta potential, the particles are kept apart and will remain in stable suspension. If, on the other hand, the radius of diffusion of the outer layer is reduced to the point where the van der Waals forces prevail, the particles are attracted to form agglomerates which tend to separate from the liquid phase.

Extending this principle to the present invention: a dense layer at the surface of the inner pore walls of the nanoporous membrane, consisting of ions in the electrolyte solution attracted by the surface charge present at the surface of the inner pore walls of the nanoporous membrane, exhibits a charge of polarity opposite to the natural charge of the nanoporous membrane itself under the physical and chemical conditions of the electrolyte solution. An outer layer of opposite polarity, also consisting of ions in solution, is diffused within a given distance from the surface of the nanoporous membrane. The net potential between the two layers is referred to as the zeta potential. The zeta potential is the potential difference between the dense layer of Stern and the liquid. It therefore characterizes the distribution of electrical charges on the surface of the inner pore walls of the nanoporous membrane.

As such, the zeta potential depends on the ionic force of the electrolyte solution, and concentration in ions in solution, around the membrane surface.

To increase the zeta potential and improve the reverse osmotic flow generated on either side of the reverse osmotic membrane element, the pH of the electrolyte solutions (11A) and (11B) may be adjusted as a function of the isoelectric point of the inner surface of the nanochannels of the nanoporous membrane bearing a surface charge (32) (composite membrane element) or (32A) (hybrid membrane element).

The effect of pH on the membranes surface charge may be studied by measuring the zeta potential of the nanoporous membrane samples at different pH of the electrolyte solution. The zeta potential of nanoporous membranes may be pushed towards negative values with the increase in the pH values of the electrolyte solution. For example, one mechanism by which pH increase drives the zeta potential towards negative values includes the deprotonation of species at the surface of the membrane inner pore walls (e.g., deprotonation of OH to $O^-$, for example on $TiO_2$ or $SiO_2$ membrane surface).

For example, when ceramic nanoporous membranes, such as $TiO_2$ or BN nanoporous membranes are used, to obtain a negative charge on the inner surface of the nanochannels, the pH of the solutions can be adjusted to a value of between (pHiso+1) and 14, more favourably between the values (pHiso+2) and 12. To obtain a positive charge on the inner surface of the nanochannels, the pH of the solutions can be adjusted to a value of between 0 and (pHiso-1) further favourably between 1 and (pHiso-2). The increase in the negative zeta potential values of nanoporous membranes along with the the pH increase may occur due to the deprotonation of the functional groups on the membrane surface, for example.

As used herein, "pHiso" refers to the pH of the isoelectric point of the constituent material of the inner surface of the nanochannels. pHiso is measured using methods known to skilled persons, in particular with the potentiometric acid-base titration method.

When the solute-containing electrolyte solution is an aqueous solution, the aqueous solution may have a pH of 2 to 13, depending on the nature of the nanoporous membrane and the surface charge naturally present on it, and a temperature at which water is maintained in a liquid state, for example, 0 to 100°C, preferably 15 to 50°C, more preferably 20 to 40°C. The temperature may be higher or lower than the above temperature. For example, a mixture of other solute/solvent may have a temperature deviating from the above temperature.

Electrodes (20A) and (20B)

As discussed previously, each of the vessels (10A) and (10B) of the purification/filtration system according to the invention comprises an electrode (20A and 20B, respectively) arranged so that the electrode comes in contact with the electrolyte solution (11A and 11B, respectively). Different types of electrodes may be used in the context of the present invention. All types of electrodes capable of collecting the flow of cations or anions (e.g, $Na^+$ or $Cl^-$ ions) may be used, preferably electrodes composed of silver and silver Chloride (Ag/AgCl), Carbon and Platinum (C/Pt-), Carbon (C-), Graphite or Iron complexes of the type $[Fe(CN)_6]^{4-}/[Fe(CN)_6]^{3-}$. Advantageously, the first and second electrodes (20A) and (20B) may be platinum electrodes.

The electrodes (20A) and (20B) can be partly or fully immersed in the electrolyte solutions (11A) and (11B), respectively. Provision could also be made so that the electrodes (20A) and/or (20B) may be in the form of at least one portion of a wall of the vessels (10A) and/or (10B). These electrodes (20A) and (20B) are both connected to an electrical source (40) allowing the generation of an electric field between both electrodes. These electrodes may be connected via mere cables to the electrical source (40).

Electric energy source element (40)

In the system and process according to the invention, the electric energy source element (40) may be any suitable electrical source known in the art. For example, it may be a power generator, a battery, a photovoltaic pannel, or any other form of electrical source.

Advantageously, the electric energy source element (40) may comprise one or more batteries. As such, the reverse electro-osmotic filtration system according to the present invention may be a portable/mobile system. For example, the electric energy source element (40) may be configured to be charged by means of light, wherein particularly the electric energy source element comprises a solar cell or a photo diode.

Advantageously, the electric energy source element (40) may be configured to be charged by means of using the reverse electro-osmotic effect of pumping electrolyte solution through the reverse osmosis membrane element (30). To that effect, the electric energy source element (40) may comprise a hydro turbine element operatively connected to the reverse electro-osmotic membrane element (30).

Flow rate control (50)

The reverse electro-induced flow rate through the composite or hybrid membrane element according to the invention may be controlled using different parameters:

(i) Surface charge of the nanoporous membrane: the flow rate across the membrane element may be increased with membranes having a higher surface charge. The surface charge may be adjusted with the choice of the material, any chemical modification that may be applied to modify the membrane surface charge, the pH of the electrolyte solution in contact with the membrane surface bearing a surface charge. Advantageously, a minimum surface charge with a |zeta potential| $\geq 5$ mV is preferred, more preferably $\geq 20$ mV, most preferably $\geq 50$ mV. The membrane surface charge may be adjusted based on the choice of the material making up the membrane and/or based on the ionic force/concentration of the electrolyte solutions in contact with the membrane, as described previously.

(ii) Pore size: With respect to the semipermeable membrane (e.g., when a composite membrane is used), the pore size will naturally depend on the size and/or the chemical characteristics of the solute to be filtered/separated. With respect to the nanoporous membrane element (which fulfills the "pumping function" in the system), there are no particular constraints concerning pore size except in cases where the nanoporous membrane element also fulfills the role of size-exclusion-type semi-permeable membrane (as is the case for example for hybrid membrane elements described herein, in which case to nanoporous membrane porosity should be adapted to the size of the solute to be separated/filtered out). In other words, electro-osmosis has no or little dependence on the nanoporous membrane element pore size. The nanoporous membrane pore size just needs to be sufficient to induce the electro-osmotic flow of the polar solvent through the membrane. This is true for variants where a composite membrane is used, and also variants where a hybrid membrane is used (i.e., when a dual semi-permeable/nanoporous membrane element is used, such as in Figure 2). Advantageously, the porosity of the nanoporous membrane element (which fulfills the function of electro-inducing the flow of the polar solvent in the system) may be < 500 nm, preferably < 300 nm, more preferably < 100 nm, most preferably < 50 nm. In a preferred variant, the filtration system is intended for ultrafiltration, and the porosity of the nanoporous membrane element may be between 1-50 nm.

(iii) Pore geometry: in general, semi-permeable membranes may not have a well-defined pore geometry (it can be "spaghetti-like for example). With respect to nanoporous membrane elements, there are no particular constraints concerning pore geometry: the system will work with any pore geometry (provided that the nanoporous membrane material bear a suitable surface charge, as further detailed herein), ranging from materials with symmetrical pore shape (such as anodic aluminum oxide, which features cylindrical pores) to materials having highly tortuous porosity (such as polycarbonate). For example, porosities with nanotubular morphology i.e. cylindrical pores of circular cross-section, conical asymmetric pore shapes, honeycomb pore geometry, hourglass-shaped porosity, etc.. may be used.

The electro-osmotic flow across the nanoporous membrane may be optimized by modulating the pore geometry of the membrane. This may be achieved empirically by changing the pore geometry of the given nanoporous membrane, using methods well known in the art.

generally the flow rate may be optimized if membrane pore geometry is preferably symmetrical and has little to no tortuosity. As such, nanotubular morphology i.e. of circular cross-section will have the effect of increasing flow rate across the membrane, as opposed to conical asymmetric pore shapes for example.

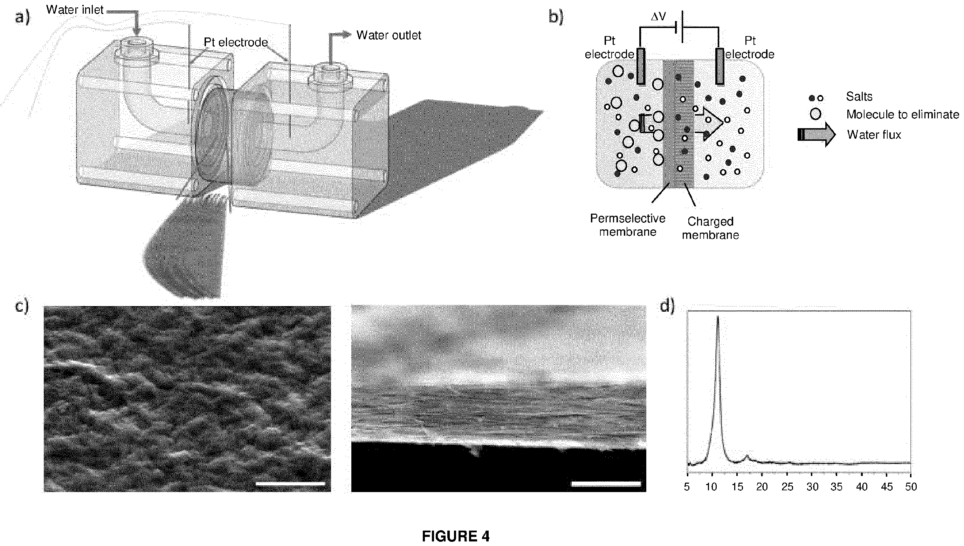

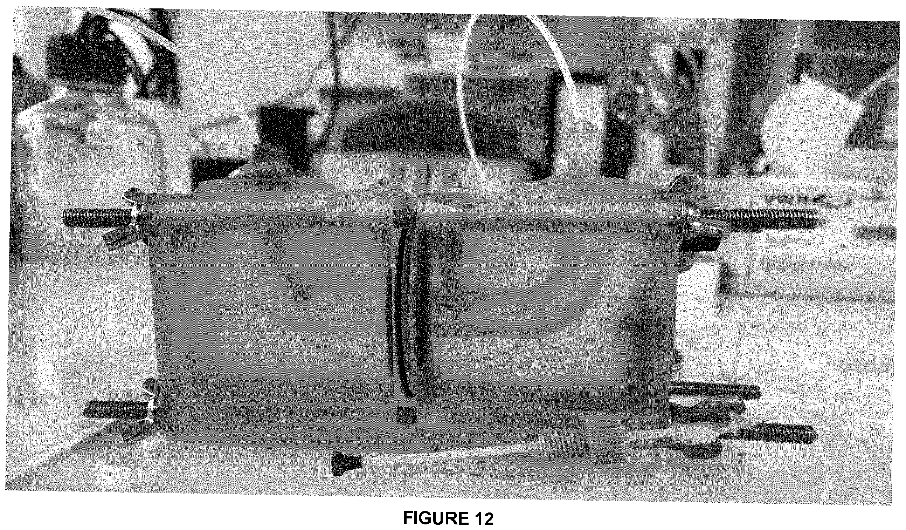

(iv) Electrical field intensity: for a given nanoporous membrane (32) and membrane surface area exposed to the electrolyte solution in the system according to the present invnetion, the flow rate increases as the applied current (hence the electrical field) between the electrodes increases (cf. Figures 4B and 12 for example). The choice of the applied current will depend in part on the type of filtration application (microfiltration (50-500 nm), ultrafiltration (1-50 nm), or nano-filtration ($\leq$1 nm)). Intensity and electric field are chosen to maximize the flow. It will also depend on the number of pores through which the electro-osmotic flow can be induced, hence the surface area of the nanoporous membrane in contact with the electrolyte solution and the surface pore density of the nanoporous membrane. Generally, flow versus intensity (or current) curves are plotted for given conditions of pH concentration etc... in the electrolyte solution, to determine the optimal range of electrical field intensity. For ultrafiltration purposes, the applied current preferably ranges from 0.5 mA to 20 mA, for example from 0.5 mA to 15 mA, from 0.5 mA to 10 mA, preferably from 0.5 mA to 5 mA, more preferably from 0.5 mA to 2 mA, relative to the membrane size. The electrical field intensity will preferably be adapted depending on the electrode and/or membrane used : caution should be exercized that there are no secondary red/ox reactions that can damage the electrode and/or the membrane, for example. For example, when platinum electrodes are used, there is no limit in the voltage that can be applied *per se* (the electrodes can withstand any voltage) : the voltage limit will rather lie in the voltage threshold where solvent electrolysis starts (for example, when water is used as solvent, the applied voltage should not go beyond 5V to avoid water electrolysis). Conversely, when Ag/AgCl electrodes are used, the applied voltage should not go beyond 1V to avoid secondary red/ox reactions at the electrode level (in this case, it is the nature of the electrodes that will limit the voltage to be applied).

(v) Ionic force/concentration of the electrolyte solution: the flow rate is inversely proportional to the salt concentration in the electrolyte solution (cf. Figures 4B and 12 for example). The choice of the salt concentration in the electrolyte solution will depend on the flow rate that is to be achieved. For ultrafiltration purposes, the salt concentration in the electrolyte solution preferably ranges from 0.1 mM to 100 mM, preferably from 0.1 mM to 50 mM, more preferably from 0.1 mM to 10 mM, most preferably from 1 mM to 10 mM.

In summary, using a composite membrane with nanometric porosity (<500 nm), which bears a significant surface charge, we have developed a filtration method based on the application of an electric field. When said membrane separates two reservoirs containing water (or other polar solvent) and mobile electrolytes (e.g. salts), the application of an electric field between the two reservoirs of the cell generates an electro-osmotic flow (movement of water/solvent from one cell to the other, in the direction opposite that of normal osmotic flow). It has been demonstrated herein that it is possible to use this reverse osmotic flow for filtration/purification applications by the unexpected combination of a nanoporous membrane with a surface charge and a selective membrane. Thus, water charged with electrolytes can be used as a separation vector for uncharged molecules (hormones, dyes, drugs, etc.). It is not necessary to have a difference in electrolyte concentration between the two reservoirs of the cell, nor is it necessary to apply a difference in pressure to obtain a flow. This flow can be controlled by different parameters, such as the surface charge of the membrane, the diameter of the pores and their geometry (tortuosity, asymmetry...), and the electric field.

As discussed previously, the scarcity of drinking water resources is a major international concern. On the one hand, certain industrial wastes, particularly pharmaceutical and chemical, pose major problems for treatment plants. On the other hand, some arid countries massively exploit reverse osmosis desalination processes to obtain drinking water. However, the energy cost of these processes greatly increases the price of water, but also of the installations.

Electric field filtration makes it possible to dispense with the application of high pressures in traditional water treatment processes. For example, for desalination, it is necessary to apply pressures exceeding the osmotic pressure (>30 bars). These processes are expensive and complex to implement. The application of an electric field reduces water treatment costs even for larger molecules (hormones, drugs...) which are sometimes difficult to eliminate. Combining the use of 2D nanomaterials (such as graphene oxide or boron nitride) with this filtration method also makes it possible to work with thinner membranes, thus improving permeability for identical selectivity.

The present invention reduces to practice the use of electric field to induce separation and filtration, contrary to standard methods (typically reverse osmosis type) based on mechanical forcing by pressure difference. Therefore, the present invention offers a filtration method that is not based on a concentration difference (such as direct osmosis) or a pressure difference (reverse osmosis), but on the application of an electric field. This provides a huge economic advantage: less expensive installations, cheaper to apply electricity than pressure, process avoiding mechanical stress (which usually uses pressures of the order of 50 bars on the membranes, which is not the case here). This also has a significant ecological advantage, since it could provide an upstream solution for the filtration of complex molecules to be eliminated in purification plants. For example, an electric field filtration system according to the present invention could be installed in plants in the pharmaceutical or textile industry, for waste-water pre-treatment.

The present invention therefore provides an extremely valuable alternative for filtration/purification processes and water treatment processes, which overcomes the drawbacks of existing processes.

## EQUIVALENTS

The representative examples that follow, together with the appended Figures, are intended to help illustrate the invention, and are not intended to, nor should they be constructed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

## EXAMPLES

The process and systems according to the present invention and their reduction to practice can be understood further by the examples that illustrate how some of the processes may be carried out. It will be appreciated, however, that these examples should not be construed to limit the invention. Variations of the invention, now known or further developed, are considered to fall within the scope of the present invention as described herein and as hereinafter claimed.

## ABBREVIATIONS

GO: graphene oxide
PC: polycarbonate
PMMA: Methyl polymethacrylate

## MATERIALS AND METHODS

Commercial Graphene Oxide water dispersion of the concentration of 0,4 wt% was purchased from Graphenea SA. Potassium Chloride (>99%), Tris(2,2'-bipyridyl)dichlororuthenium (II) hexahydrate (99.95%) and testosterone (>99.9%) were purchased from Sigma-Aldrich.

All experiments were carried out at room temperature (25 $\pm$ 3°C) and at neutral pH.

### Example 1 - Composite membrane preparation and characterization

GO/PC membranes were prepared by vacuum filtration. In a typical preparation, diluted dispersion of Graphene oxide (GO) is prepared by first centrifuged at 2000 rpm the commercial dispersion provided by Graphenea SA and discard the precipitate until the density of the dispersion is adjusted to 1.003 g mL$^{-1}$. Then 1.5 mL of this suspension was diluted up to a total volume of 15 mL with MiliQ® water obtained in an advantage A10 system (Millipore). This diluted dispersion of GO was added dropwise on top of the polycarbonate (PC) substrate (Nuclepore Track-Etch Membrane, 25 MM 0.1 microns) disposed on a Büchner funnel: one end was sealed and the other end was connected to a vacuum pump. When the vacuum filtration ended, typically less than 16 hours, the composite GO/PC membrane disc was divided in two. One fraction was plugged to the purification device meanwhile the other was kept for characterization.

The surface charge of the PC membrane was measured using the conductance equation [1], and was determined to be around 0.2 mC/m$^2$.

The pore size of the GO membrane was measured using XRD, and was determined to be 0.8 nanometer on average (average interlayer distance between stacked GO flakes). The XRD diffractogram revealed a wide distribution of interlayer distances (from 0.7 to 1.4 nm).

Materials characterization

The surface morphology of the GO/PC membranes was observed with an optical Microscope in transmission mode and by a scanning electronic microscope (Thermo-Fischer™ Quattro S). X-Ray diffractograms of the membrane were collected in refraction mode by using a powder XRD diffractometer (Bruker®). Nitrogen adsorption and desorption isotherms at 77 K were collected in a TRIFLEX micrometrics® volumetric instrument. The 00I peak of the recorded diffractogram was used to determine the average interspace between GO layers in GO/PC membranes, meanwhile the pore size distribution of the membrane and its specific surface area was determined by applying a DFT model and BET to the typical nitrogen adsorption isotherm.

## Example 2 - Filtration of a dye complex

The evaluation of electro-osmotic purification system according to the invention was performed using a custom electrokinetic set-up where a composite GO/PC membrane prepared as in Example 1 was separating two reservoirs of 6 mL volume each (set-up of Figure 1). Each reservoir (10A) and (10B) was filled with a solution of water and the same concentration of salt (KCl) during flow rate determination. For rejection rate experiments, from 1 to 200 $\mu$M of Tris(2,2'-bipyridyl)dichlororuthenium (II) hexahydrate (99.95%) (Ru-bypi) was added as test pollutant in reservoir (10A).
One Pt electrode (Sigma-Aldrich) was placed in each reservoir (10A) and (10B) and was wire connected to a keithley 2410 sourcemeter to ensure constant tension between -1 and 1 mA.
The interspace between the two reservoirs was equipped with a PMMA disk with a round hole of 0.5 cm diameter in the middle. To that effect, glue (Staycast 1266, Aldrich) was spread around the GO/PC composite membrane, letting free the middle section that faces the hole on the PMMA disk. After curing of the glue (typically 24 hours), the PMMA disk with the GO/PC composite membrane covering the hole was placed between the reservoirs, rested on each O-rings and then pressed and mechanically sealed.
After closing the electric circuit, the flow rate from reservoir (10A) to reservoir (10B) was measured by coupling outlet pipes of the reservoirs to a flowmeter, that consists in a milimetric calibrated capillary: briefly, each reservoir was independently connected to the flowmeter (a glass capillary of a known diameter with a ruler next to it so that the volume can be known). The quantitative determination of the Ru-bypi was performed by UV-Vis in a nanodrop (Thermo Fisher Scientific). The external calibration curve of the instrument and the rejection measurements were performed by preparing 0.5 $\mu$M to 100 $\mu$M solutions of Ru(bypi) and determine the absorption capacity at 283 nm.

## Example 3 - Filtration of a hormone

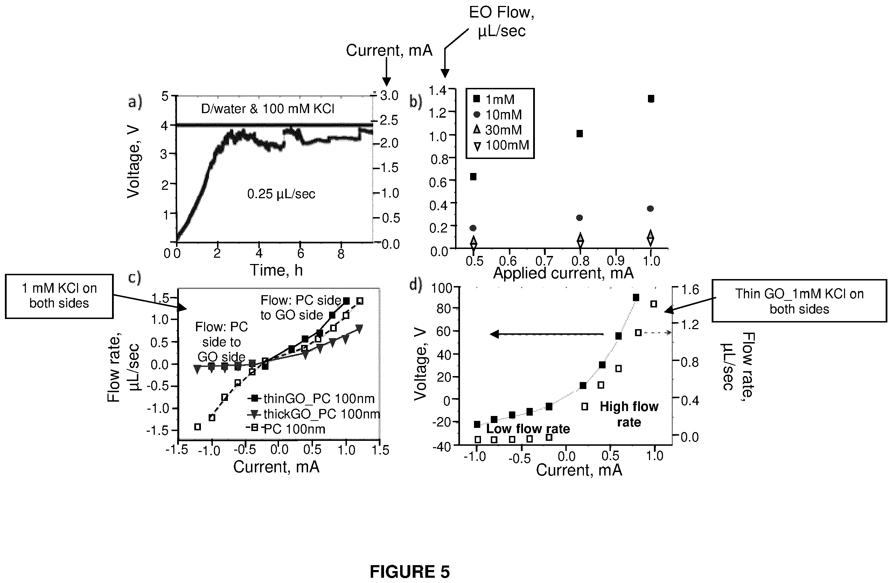

Example 2 was repeated, using 10 mg mL$^{-1}$ testosterone as pollutant, instead of the Ru(bypi) dye.
The quantitative determination of testosterone was performed by UV-Vis in a nanodrop (Thermo Fisher Scientific). The external calibration curve of the instrument and the rejection measurements are performed by preparing 0.9 $\mu$M and 9 $\mu$M solutions of testosterone and determine the absorption capacity at 283 nm (cf. Figure 5A).

RESULTS AND DISCUSSION

Through the Examples that precede, an ultrafiltration process and system has been achieved by way of a thin composite semipermeable membrane (~8 $\mu$m), composed of Graphene Oxide (semipermeable membrane) in association with a polycarbonate nanoporous membrane which presents a suitable surface charge, where the flow is obtained by applying a low energy electric force. The Examples illustrate an experimental set-up consisting in two reservoirs (10A) and (10B) separated by a composite membrane according to the invention where a voltage drop can be applied between the faces of the membrane. The system was tested in terms of flux and rejection rate, initially with a probe molecule to optimize the operative conditions and finally a pollutant that it is often contaminating drinkable water, testosterone hormone.
Without wishing to be bound by any particular theory, it is proposed that under a voltage drop, an electro-osmotic flow of water is generated within the nanopores of the PC membrane due to the Debye layer formed in close contact with the charged surfaces of the PC material. The overall asymmetry of the membrane, made of a nanoporous material that exhibits surface charge [2] leads to an asymmetric response of the water flow versus the voltage drop. The flow rate of water takes the expression

$$Q = Qosm + Qs * (exp(V/V0) - 1) \qquad \textbf{(Equ. 2)}$$

where Qosm = K_hydro * kT Dc*2 is the osmotic contribution to the flow rate and the second term originates from the rectified electro-osmotic flow in the asymmetric membrane; Qs is the saturating value for the flux and depends on the characteristics of the membrane pores.

Due to the semipermeable nature of the GO membrane, unable to carry the ions, only water is allowed to cross the GO/PC membrane. Meanwhile, the high cohesion of the layer by layer GO membrane provides high selectivity during the purification process.

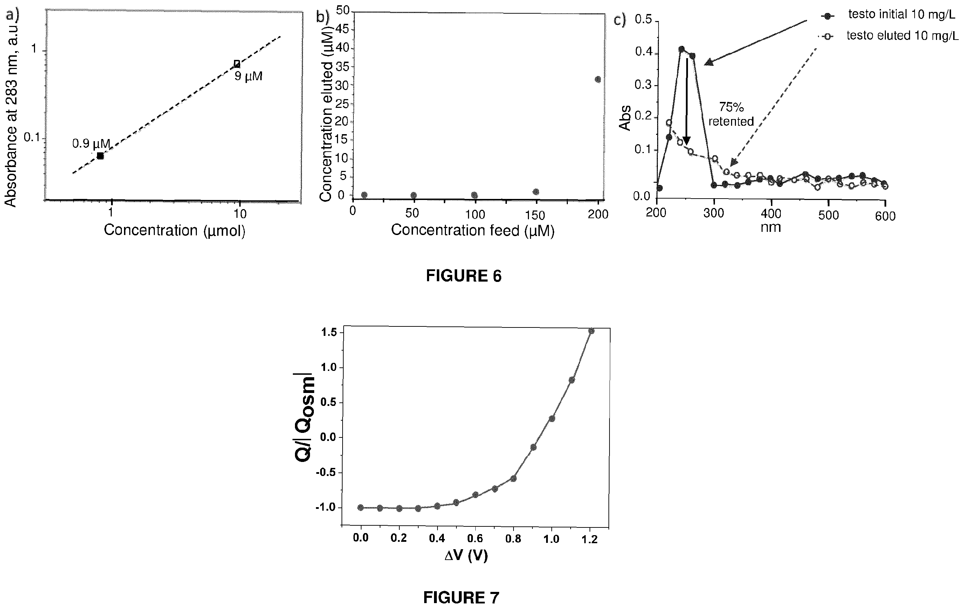

First, the water flow throughout an asymmetric membrane under a voltage drop ($\Delta V$) was computed (Figure 7). Interestingly, for $\Delta V>0$ the flow increases exponentially with voltage drop and overcome the osmotic contribution of the flow. This shows that the osmotic flow rate can be counterbalanced above a threshold voltage. In this situation a flow can be produced from the positive to the negative pole of the membrane.

At this stage it is noteworthy to remark that the equation above shows that the characteristic voltage in Eq. 2 is fixed by $\Delta V_0$.

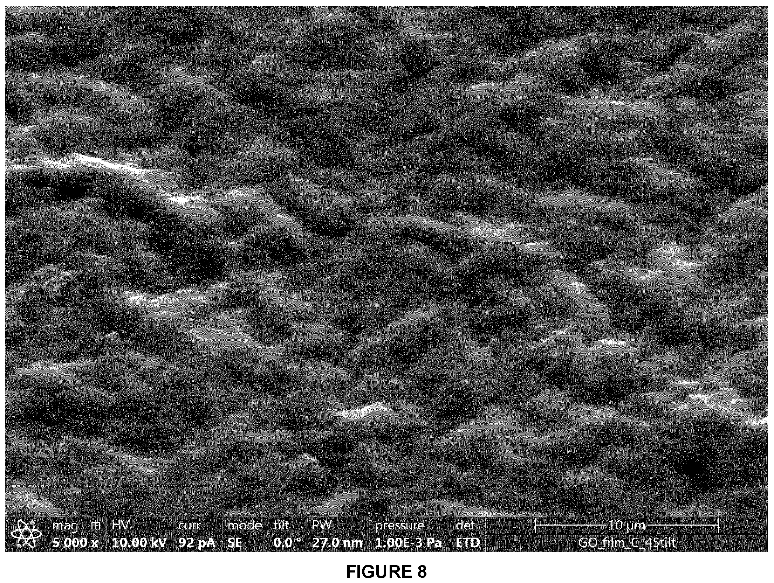

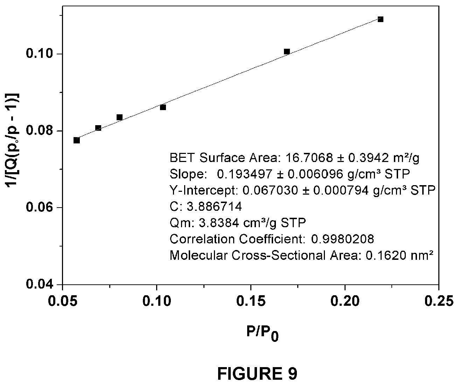

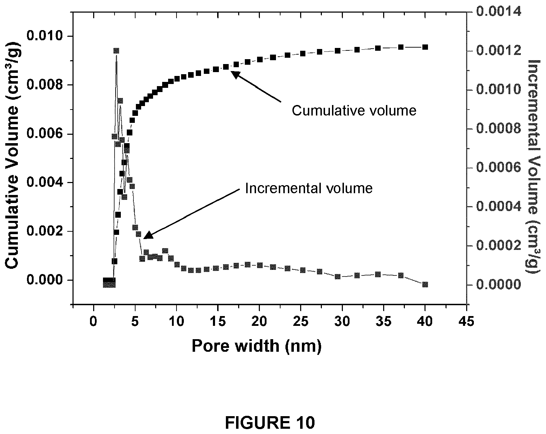

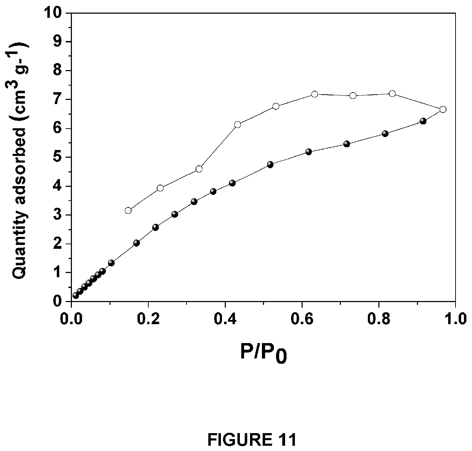

Then, an easy scalable device was prepared, that is capable to operate under these premises for exploiting the phenomena in water purification from pollutants that represent an incoming environmental and human health hazard. This device consisted in two reservoirs (10A) and (10B) separated by a semipermeable GO/PC membrane prepared in Example 1, that were further filled with a solution of water and salt (KCl) in each and then coupled to an electric source by two platinum electrodes (Figure 4A). The prepared membrane GO/PC, suitable for purification since no cracks are detected in long range (Figure 8), was formed by the stacking of flakes of GO, layer by layer, with the aid of pressure difference and water permeation. FE-SEM images of the surface of the membrane (Figure 4B) confirm their previous observed cohesion, meanwhile the cross-section of the membrane reveals the densely interlayer structure of the membrane with a thickness of ca. 8 micron (Figure 4C). This membrane presents an average interlayer distance of 0.8 nanometer as derived from the PXRD diffractogram (Figure 4D). The broad peak recorded in the diffractogram points to a wide distribution of interlayer distances (from 0.7 to 1.4 nm), which is one of the desirable conditions for our asymmetric membranes. **[2]** The presence of the PC substrate is also confirmed in the diffractogram. **[3]** The further analysis of the porosity of the membrane was performed in the absence of the PC substrate and reveals an apparent surface area of 18 $m^2g^{-1}$ (Figure 9) in agreement with previous reported GO membranes prepared by following a similar approach **[4]**. The main pore size distribution derived for the collected data shows a wide distribution from 1.8 nm to 6 nm (Figure 10), which in addition to the fact that the isotherm trend to achieves a plateau around 0.6 $P/P_0$ (Figure 11) points to the absence of big cavities in the membrane, in good agreement with the previously observed in the intended microscopies performed.

Once the membrane of GO was conformed, the GO/PC membrane was placed between the reservoirs of the system. The contents of the reservoir (10A) was in contact with the GO side of the composite GO/PC membrane, while the contents of the reservoir (10B) was in contact with the PC side of the composite GO/PC membrane. The electrokinetic cell was mechanically closed and several experiments were assayed to demonstrate its feasibility for the phenomena and the intended application.

Each reservoir was filled independently with a 6 mL of water to confirm first the absence of leaks in the system and then the stationarity of the flux if no electric driving force was applied (Figure 12). A voltage was then applied by fixing 1 A to the electrodes. As expected, after 2 hours, no change in the system was detected since the driven force is only given by the movement of the ions that counterbalance the GO surface charge close to the surface. **[5]**

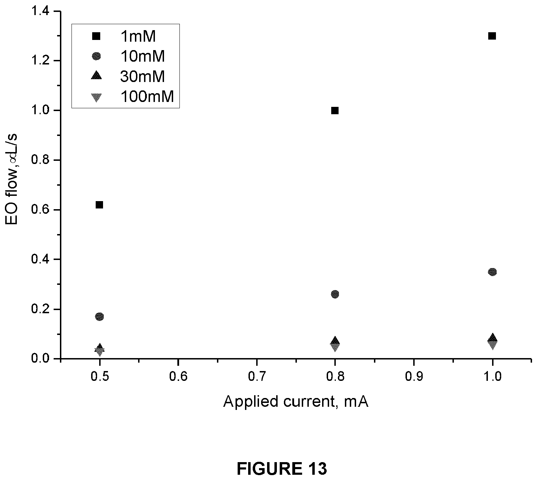

Once a second agent was included, that is capable to create these ion imbalance (100 $\mu$M of KCl), a water flux was detected after applying the driving force (Figure 5A). The boundary conditions of the phenomena was estimated at $\Delta V$ = 0 as predicted by our theoretical model. Then, the flux obtained at different salt concentrations was estimated and intensity applied by using the GO/PC membrane (Figure 5B) or by using the bare PC substrate as reservoir separators (Figure 13). Figure 5B reveals a clear trend, the flow throughout the system was increased as the concentration of salt was decreased. This is observable in both membranes, bare PC and GO/PC for the concentration range of salt from 100 mM to 1 mM and an intensity between 1 mA and 0.5 mA. Thus, an operative condition of 1 mM of KCl was fixed. As no additional pressure is required to drive the flux throughout the membrane, the use of thin membrane is preferred in term of cost, since less amount of starting material is required.

A variable intensity was applied to the electrodes ranging from -1 to 1 A (Figure 5C). The water flux performed between no flux and 1.3 $mLs^{-1}$ in an exponential correlation over the threshold with the intensity applied and with the absence of flux below this value, in good agreement again with the model prediction. Interestingly, the negative nature of the GO surface is also rebelled by the absence of this flux when at $\Delta V < 0$. Under these conditions no interphase close to the surface can be formed and as consequence no flux is promoted. It has to be also noted that the resilience of the membrane to the process was demonstrated since no flux variation was obtained if the determination was performed by keeping same membrane and changing the feeding solution or by switching to a fresh one. Once the base of the phenomena was established, the voltage drop of the interphase during the process was monetarized (Figure 5D). The obtained values suggest a low cost energetic process comparable to pressure driven processes. If the lab scale values are extrapolated, and considering a linear correlation, without further optimization, we estimate that for a membrane window of 1 $m^2$ the flux thorough the membrane will be over 238 L per hour.

Illustration of device for purification under electro-osmotic conditions

Under the conditions described above, only water molecules flow when the voltage drop is applied, meanwhile the interlayer stacking of the GO membrane acts as a molecular sieve for molecules above the hydrodynamic diameter of the membrane porosity, providing the selectivity of the membrane.

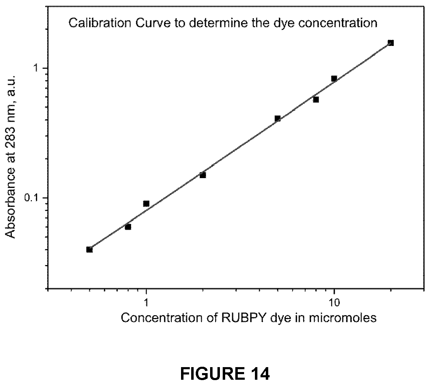

For rejection experiments, a small molecule (Ru-bipy) was selected, with an expected hydrodynamic radius of 0.6 nm. **[6]** The rejection rate of the molecule was determined after 2 hours of electro-osmotic process (Equ. 3, Figure 14).

$$\text{Rejection} = 1- (\text{Cperm}/\text{Cfeed}) \textbf{ (Equ. 3)}$$

where Cperm represents the concentration of solute in the permeate, and
Cfeed represents the concentration of solute in the feed solution (the electrolyte solution that is meant to be filtered using the system).

In a first assay, Ru-bipy filtration was performed at 9 $\mu$M of feed (Ru-bipy concentration) and constant intensity of 1 mA obtaining a promising rejection of 90% (Figure 6A). This experiment already demonstrated the use of the system according to the invention to purify water that contain small molecules, under reverse electro-osmotic conditions.

Full rejection assay

The intensity applied was decreased to 0.5 mA, thereby reducing the applied driving force and as consequence the probability of the molecule to cross through the GO membrane nanodefects. Under these conditions, full rejection was obtained with Ru-bipy up to 150 $\mu$M where the rejection decrease to 0.99, and finally 0.84 when 200 $\mu$M Ru-bipy solution is fed respectively (Figure 6B). This full rejection can be obtained under low energetic cost conditions.

Hormone rejection

The reservoir (10A) containing the electrolyte solution in contact with the GO side of the composite GO/PC membrane, was filled with a water solution of the hormone testosterone (10 mg mL), which means over 100 times the values expected in real water samples. **[7]** This molecule commonly present in several water sources is difficult to detect and filtrate due to its small ratios.

The optimized conditions were applied (intensity applied was decreased to 0.5 mA, for the membrane used in the Examples, ~78 mm$^2$ surface area, corresponding to the surface of the interspace between the two reservoirs (10A) and (10B): 0.5 cm diameter disk) and a retention of ca. 75% of the hormone present in the feed solution (Figure 6C) was obtained, showing the huge potential of this new approach for water purification.

In summary, we have developed an experimental set up for water purification by reverse electro-osmosis composed by a feeding reservoir (10A), a semipermeable graphene oxide-based membrane, an eluted reservoir (10B) and two electrodes to induce voltage drop between the two faces of the membrane. This experimental set-up is capable to achieve a water flux of 4.7 ml/h. Considering its small size and that the phenomena is expected to be linearly extrapolated, we consider that a bigger membrane section will raise comparable flux to traditional methods of filtration, but with the advantage of the high selectivity of the approach according to the present invention. Reverse electro-osmosis purification performed with a GO based membranes has been optimized in terms of energy consumption and rejection estimating a threshold voltage V>0 to produce the flow, and a full rejection of a test molecule (Ru-bipy) of hydrodynamic radius of 0.6 nm when 0.5 mA are applied. Finally, the device was also tested for applications in the filtration of hormones (testosterone) over the common concentration nowadays detected in drinkable water, estimating a high rate of retention (75 %) with no significant loss of flow detected.

**LIST OF REFERENCES**

**1.** Bocquet, L. & Charlaix, E. Nanofluidics, from bulk to interfaces. Chem. Soc. Rev., 2010, 39, 1073-1095.
**2.** Zhang et al. JACS, 2015, 137, 46, 14765-14772.
**3.** Vijayalakkshmi et al. International Journal of Polymer Science, 2011, 1687.
**4.** Wei et al. International journal of hydrogen energy, 2016, 41, 11692-11699
**5.** Kirby, 2009, Cambridge University press. Micro-nanoscale fluid mechanics: Transport in microfluidic devices.
**6.** Joshi et al. Science, 2014, 343, 752-754.

**7.** US geological survey (USGS), 2008, www.epa.gov

**8.** A. Siria, P. Poncharal, A.-L. Biance, R. Fulcrand, X. Blase, S. Purcell, L. Bocquet, "Giant osmotic energy conversion measured in a single transmembrane boron-nitride nanotube" Nature 494 455-458 (2013)

**Claims**

**1.** A reverse electro-osmotic filtration system comprising:

a) a first vessel (10A) intended to receive an electrolyte solution (11A) <u>concentrated</u> in a solute of interest, and comprising a first electrode (20A) in contact with the electrolyte solution (11A) contained in vessel (10A);

b) a second vessel (10B) intended to receive an electrolyte solution (11B) <u>substantially free</u> of, or <u>depleted</u> in, the same solute of interest, and comprising a second electrode (20B) in contact with the electrolyte solution (11B) contained in vessel (10B);

c) a reverse osmosis membrane element (30) separating the two vessels, combining

(i) a semipermeable membrane element (31), and

(ii) a nanoporous membrane element (32) having a surface charge with a |zeta potential| $\geq$ 5 mV, preferably $\geq$ 20 mV, most preferably $\geq$ 50 mV;

wherein the semipermeable membrane element (31) and the nanoporous membrane element (32) having a surface charge may be one and the same element, or two distinct elements;

wherein the semipermeable membrane element (31) is configured to be in contact with the electrolyte solution (11A) of the first vessel (10A), and the

said reverse osmosis membrane element (30) fulfilling both (i) a function of solute filtration and (ii) a function of electroinducing the flow of the solvent from the electrolyte solution (11A) from vessel (10A) to vessel (10B) through the reverse osmosis membrane element (30);

the electrolyte solutions (11A) and (11B) in vessels (10A) and (10B), respectively, comprising the same polar solvent;

the first and second electrodes (20A) and (20B) being operatively coupled to an electric energy source (40);

wherein the application of an electric field between the first and second electrodes (20A) and (20B) induces a reverse-osmosis flow of the polar solvent of the electrolyte solution (11A) from vessel (10A) to vessel (10B), through the reverse osmosis membrane element (30).

**2.** The reverse electro-osmotic filtration system according to claim 1, wherein the semipermeable membrane element (31) and the nanoporous membrane element (32) having a surface charge are two distinct elements combined together to form a two-layer composite asymmetric membrane (30A), said composite asymmetric membrane (30A) comprising a semipermeable membrane (31) superimposed with a charged nanoporous membrane (32) bearing a surface charge with a |zeta potential| $\geq$ 5 mV, preferably $\geq$ 20 mV, most preferably $\geq$ 50 mV.

**3.** The reverse electro-osmotic filtration system according to claim 2, wherein the semipermeable membrane (31) is a size exclusion membrane, an ion exchange membrane, or any other membrane allowing the separation/filtration of particular molecules or ions from a given electrolyte solution (for example semi-permeable membranes based on separation by chemical affinity), preferably a size exclusion selective membrane or an ion exchange membrane.

**4.** The reverse electro-osmotic filtration system according to claim 3, wherein the semipermeable membrane (31) is a size exclusion selective membrane composed of stacked graphene oxide flakes.

**5.** The reverse electro-osmotic filtration system according to claim 2, wherein the nanoporous membrane (32) bearing a surface charge is essentially formed of a material selected from $TiO_2$, boron nitride, $SiO_2$, polyethersulfone, polycarbonate, anodic aluminum oxide, hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes; preferably $TiO_2$, BN, $SiO_2$, polycarbonate, anodic alumina, hydrotalcite, Ni-Fe layered double hydroxide, $Ni_2$dobdc, $Mg_2$dobdc, cellulose or polyelectrolyte layers polymer membranes; most preferably $TiO_2$, BN, anodic alumina, $SiO_2$, or polycarbonate; and having a porosity < 500 nm, preferably < 300 nm, more preferably < 100 nm, most preferably < 50 nm.

6. The reverse electro-osmotic filtration system according to claim 2 or 4, wherein the surface of the charged nanoporous membrane (32) is chemically modified to enhance the nanoporous membrane surface charge.

7. The reverse electro-osmotic filtration system according to claim 6, wherein the chemical modification is effected on the surface of the nanoporous membrane pore walls.

8. The reverse electro-osmotic filtration system according to any one of claims 5 to 7, wherein the charged nanoporous membrane (32) is obtained from a polycarbonate membrane having a porosity < 500 nm, preferably < 300 nm, more preferably < 200 nm; the inner pore walls of which have been chemically modified by dip-coating the polycarbonate membrane in an aqueous solution of polydopamine.

9. The reverse electro-osmotic filtration system according to claim 1, wherein the semipermeable membrane element (31) and the nanoporous membrane element (32) having a surface charge are one and the same element to form a single hybrid membrane (30B), said single hybrid membrane (30B) comprising a porosity < 500 nm, preferably < 300 nm, more preferably < 100 nm, most preferably < 50 nm, and a surface charge on the hybrid membrane inner pore walls surface with a |zeta potential|$\geq$ 5 mV, preferably $\geq$ 20 mV, most preferably $\geq$ 50 mV.

10. The reverse electro-osmotic filtration system according to claim 9, wherein the hybrid membrane (30B) is a nanoporous membrane made of a material having a surface charge with a |zeta potential|$\geq$ 5 mV, preferably $\geq$ 20 mV, most preferably $\geq$ 50 mV, such as $TiO_2$, boron nitride, $SiO_2$, polyethersulfone, polycarbonate, anodic aluminum oxide, hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes; preferably $TiO_2$, BN, $SiO_2$, polycarbonate, anodic alumina, hydrotalcite, Ni-Fe layered double hydroxide, $Ni_2$dobdc, $Mg_2$dobdc, cellulose or polyelectrolyte layers polymer membranes; most preferably $TiO_2$, BN, anodic alumina, $SiO_2$, or polycarbonate.

11. The reverse electro-osmotic filtration system according to claim 9, wherein the hybrid membrane (30B) is a size exclusion selective membrane coated on its inner pore walls with a material having a surface charge with a |zeta potential| $\geq$ 5 mV, preferably $\geq$ 20 mV, most preferably $\geq$ 50 mV, such as $TiO_2$, boron nitride, $SiO_2$, polyethersulfone, polycarbonate, anodic aluminum oxide, hydrotalcite, Ni-Fe layered double hydroxide, $Ni_2$dobdc, $Mg_2$dobdc (dobdc = 1,4-dioxido-2,5-benzenedicarboxylate), cellulose or polyelectrolyte layers polymer membranes such as nanoporous membranes obtained by sequentially dip-coating layers of cationic polyethyleneimine and anionic poly(acrylic acid) onto polycarbonate membranes; preferably $TiO_2$, BN, $SiO_2$, polycarbonate, anodic alumina, hydrotalcite, Ni-Fe layered double hydroxide, $Ni_2$dobdc, $Mg_2$dobdc, cellulose or polyelectrolyte layers polymer membranes; most preferably $TiO_2$, BN, anodic alumina, $SiO_2$, or polycarbonate.

12. The reverse electro-osmotic filtration system according to any one of claims 1 to 11, wherein the electric energy source element (40) is a battery.

13. The reverse electro-osmotic filtration system according to claim 12, wherein the electric energy source element (40) is configured to be charged by means of one of: light, wherein particularly the electric energy source element comprises a solar cell or a photo diode; or using the reverse electro-osmotic effect of pumping electrolyte solution through the reverse osmosis membrane element (30), wherein particularly the electric energy source element (40) comprises a hydro turbine element operatively connected to the reverse electro-osmotic membrane element (30).

14. A process for purifying an electrolyte solution in a polar solvent, comprising the steps of:

i) providing an electrolyte solution (11A) comprising an undesired solute in a polar solvent;
ii) providing a second electrolyte solution (11B) in the same or different polar solvent as in step i), not comprising the undesired solute;
iii) providing a reverse electro-osmotic filtration system comprising:

a) a first vessel (10A) equipped with a first electrode (20A);
b) a second vessel (10B) equipped with a second electrode (20B); the first and second electrodes (20A) and (20B) being operatively coupled to an electric energy source (40);
c) a reverse osmosis membrane element (30) separating the two vessels (10A) and (10B), combining

(i) a semipermeable membrane element (31), and

(ii) a nanoporous membrane element (32) having a surface charge with a |zeta potential|≥ 5 mV, preferably ≥ 20 mV, most preferably ≥ 50 mV;

wherein the semipermeable membrane element (31) and the nanoporous membrane element (32) having a surface charge may be one and the same element, or two distinct elements;

wherein the semipermeable membrane element (31) is in contact with the electrolyte solution (11A) of vessel (10A), and the nanoporous membrane element (32) having a surface charge is in contact with the electrolyte solution (11B) of vessel (10B);

said reverse osmosis membrane element (30) fulfilling both (i) a function of solute filtration and (ii) a function of electroinducing the flow of the solvent of the electrolyte solution (11A) from vessel (10A) to vessel (10B) through the reverse osmosis membrane element (30);

the reverse osmosis membrane element (30) being adapted to selectively prevent the undesired solute from diffusing though the reverse osmosis membrane element (30);

iv) placing the electrolyte solution (11A) to be purified in vessel (10A), so that the first electrode (20A) with which it is equipped is in contact with said electrolyte solution (11A);

v) placing a sufficient amount of the second electrolyte solution (11B) in vessel (10B), so that the second electrode (20B) with which it is equipped is in contact with said electrolyte solution (11B);

vi) applying an electric field between the first and second electrodes (20A) and (20B) to induce a reverse-osmosis flow of the polar solvent from vessel (10A) to vessel (10B), through the reverse osmosis membrane element (30) separating the two vessels (10A) and (10B);

thereby allowing the solvent to flow from vessel (10A) towards vessel (10B), while retaining the undesired solute in vessel (10A); and

vii) collecting the purified electrolyte solution (11B) from vessel (10B), which is substantially free of, or depleted in, undesired solute.

15. The process of claim 14, wherein the polar solvent is a solvent capable of generating acidic ions, such as water, an alcohol such as methanol or ethanol, a hydroalcoholic mixture, ammonia, acetone, or acetonitrile.

16. The process of claim 14, wherein the undesired solute is selected from solid particles, organic or inorganic small molecules such as dye complexes, biomolecules such as hormones, proteins, polysaccharides, polynucleotides, polypeptides, enzymes or antibodies, pollutants, metabolic waste products, or salts/ions.

17. A water purification system comprising a reverse electro-osmotic filtration system according to any one of claims 1 to 13.

18. A water desalination system comprising a reverse electro-osmotic filtration system according to any one of claims 1 to 13.

19. An implantable artificial kidney comprising a reverse electro-osmotic filtration system according to any one of claims 1 to 13.

Exemplary system with composite membrane

FIGURE 1

EP 3 862 069 A1

Exemplary system with hybrid membrane

**FIGURE 2**

EP 3 862 069 A1

**Exemplary system with composite membrane with porous support layer**

FIGURE 3

EP 3 862 069 A1

**FIGURE 4**

FIGURE 5

FIGURE 6

FIGURE 7

EP 3 862 069 A1

| mag ⊞ | HV | curr | mode | tilt | PW | pressure | det | ————— 10 μm ————— |
|---|---|---|---|---|---|---|---|---|
| 5 000 x | 10.00 kV | 92 pA | SE | 0.0 ° | 27.0 nm | 1.00E-3 Pa | ETD | GO_film_C_45tilt |

**FIGURE 8**

FIGURE 9

FIGURE 10

FIGURE 11

FIGURE 12

FIGURE 13

EP 3 862 069 A1

FIGURE 14

Europäisches
Patentamt

European
Patent Office

Office européen
des brevets

## EUROPEAN SEARCH REPORT

Application Number

EP 20 30 5110

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/275138 A1 (SHENG PING [CN] ET AL) 7 December 2006 (2006-12-07) | 1-3,5-7, 9-19 | INV. B01D61/00 |
| Y | * paragraphs [0002], [0033], [0038], [0041], [0054], [0064], [0029], [0070]; claims; figures * | 2-8,11 | B01D61/42 B01D67/00 B01D69/12 B01D71/02 |
| X | US 2018/353906 A1 (MOTTET BRUNO [FR] ET AL) 13 December 2018 (2018-12-13) * paragraphs [0052], [0087]; claims; figures * | 1,9-19 | B01D71/50 |
| X | WO 2013/009797 A1 (UWM RES FOUNDATION INC [US]; HE ZHEN [US]) 17 January 2013 (2013-01-17) | 1,9,10, 12-19 | |
| Y | * paragraphs [0044] - [0045]; claims; figures * | 2-8,11 | |
| A | R. VALLADARES LINARES ET AL: "Higher boron rejection with a new TFC forward osmosis membrane", DESALINATION AND WATER TREATMENT : SCIENCE AND ENGINEERING ; DWT, vol. 55, no. 10, 4 September 2015 (2015-09-04), pages 2734-2740, XP055714555, UK ISSN: 1944-3994, DOI: 10.1080/19443994.2014.940220 * the whole document * | 1-19 | |

TECHNICAL FIELDS
SEARCHED      (IPC)

B01D
A61M
C02F

-/--

The present search report has been drawn up for all claims

| Place of search | Date of completion of the search | Examiner |
|---|---|---|
| Munich | 14 July 2020 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

**Application Number**

EP 20 30 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | R. K. JOSHI ET AL: "Precise and Ultrafast Molecular Sieving Through Graphene Oxide Membranes", SCIENCE, vol. 343, no. 6172, 13 February 2014 (2014-02-13), pages 752-754, XP055714120, US ISSN: 0036-8075, DOI: 10.1126/science.1245711 * page 6 * ----- | 4 | |
| Y | US 8 017 050 B2 (UNIV TEXAS [US]) 13 September 2011 (2011-09-13) * claims 1, 6 * ----- | 8 | |

**TECHNICAL FIELDS SEARCHED (IPC)**

The present search report has been drawn up for all claims

| Place of search | Date of completion of the search | Examiner |
|---|---|---|
| Munich | 14 July 2020 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 5110

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2020

| Patent document cited in search report | | Publication date | Patent family member(s) | | Publication date |
|---|---|---|---|---|---|
| US 2006275138 | A1 | 07-12-2006 | NONE | | |
| US 2018353906 | A1 | 13-12-2018 | AU | 2016313787 A1 | 12-04-2018 |
| | | | CA | 2997169 A1 | 09-03-2017 |
| | | | CN | 108367242 A | 03-08-2018 |
| | | | EP | 3344374 A1 | 11-07-2018 |
| | | | JP | 2018530866 A | 18-10-2018 |
| | | | KR | 20180057640 A | 30-05-2018 |
| | | | US | 2018353906 A1 | 13-12-2018 |
| | | | WO | 2017037213 A1 | 09-03-2017 |
| WO 2013009797 | A1 | 17-01-2013 | US | 2013017414 A1 | 17-01-2013 |
| | | | WO | 2013009797 A1 | 17-01-2013 |
| US 8017050 | B2 | 13-09-2011 | AU | 2009268532 A1 | 14-01-2010 |
| | | | BR | PI0915547 A2 | 26-01-2016 |
| | | | CA | 2767761 A1 | 14-01-2010 |
| | | | CN | 102149450 A | 10-08-2011 |
| | | | EP | 2318126 A2 | 11-05-2011 |
| | | | IL | 210488 A | 31-08-2016 |
| | | | JP | 5463355 B2 | 09-04-2014 |
| | | | JP | 2011527632 A | 04-11-2011 |
| | | | KR | 20110042307 A | 26-04-2011 |
| | | | US | 2010051538 A1 | 04-03-2010 |
| | | | US | 2010059433 A1 | 11-03-2010 |
| | | | WO | 2010006196 A2 | 14-01-2010 |
| | | | ZA | 201101059 B | 26-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUOHUA LIN ; KAIYING WANG ; NILS HOIVIK ; HENRIK JAKOBSEN.** Progress on free-standing and flow-through TiO2 nanotubes membranes. *Solar Energy Materials & Solar Cells,* 2012, vol. 98, 24-38
- **POULOMI ROY ; STEFFEN BERGER ; PATRICK SCHMUKI.** TiO2 nanotubes synthesis and applications. *Angewandte Chemistry Int. Ed,* 2011, vol. 50, 2904-2939
- **JUNG TAE PARK ; WON SEOK CHI ; SANG JIN KIM ; DAEYEON LEE ; JONG HAK KIM.** Scientific Reports. *Nature,* 2014, vol. 4, 5505
- **BOCQUET, L. ; CHARLAIX, E.** Nanofluidics, from bulk to interfaces. *Chem. Soc. Rev.,* 2010, vol. 39, 1073-1095

- **ZHANG et al.** *JACS,* 2015, vol. 137 (46), 14765-14772
- **VIJAYALAKKSHMI et al.** *International Journal of Polymer Science,* 2011, 1687
- **WEI et al.** *International journal of hydrogen energy,* 2016, vol. 41, 11692-11699
- **KIRBY.** Micro-nanoscale fluid mechanics: Transport in microfluidic devices. Cambridge University press, 2009
- **JOSHI et al.** *Science,* 2014, vol. 343, 752-754
- **A. SIRIA ; P. PONCHARAL ; A.-L. BIANCE ; R. FULCRAND ; X. BLASE ; S. PURCELL ; L. BOCQUET.** Giant osmotic energy conversion measured in a single transmembrane boron-nitride nanotube. *Nature,* 2013, vol. 494, 455-458